(19) **Europäisches Patentamt**
**European Patent Office**
**Office européen des brevets**

(11) **EP 2 908 876 B1**

(12) **FASCICULE DE BREVET EUROPEEN**

(45) Date de publication et mention
de la délivrance du brevet:
**04.01.2017 Bulletin 2017/01**

(51) Int Cl.:
***A61L 27/24*** *(2006.01)*

(21) Numéro de dépôt: **13779219.8**

(22) Date de dépôt: **18.10.2013**

(86) Numéro de dépôt international:
**PCT/EP2013/071816**

(87) Numéro de publication internationale:
**WO 2014/060568 (24.04.2014 Gazette 2014/17)**

(54) **PROTEINES RECOMBINANTES DERIVEES DU COLLAGENE A ACTIVITE DE LIAISON AU FACTEUR WILLEBRAND**

AUS KOLLAGEN GEWONNENE REKOMBINANTE PROTEINE MIT VON-WILLEBRAND-FAKTOR-BINDENDER WIRKUNG

COLLAGEN-DERIVED RECOMBINANT PROTEINS WITH VON WILLEBRAND FACTOR-BINDING ACTIVITY

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **19.10.2012 FR 1259996**

(43) Date de publication de la demande:
**26.08.2015 Bulletin 2015/35**

(73) Titulaires:
- **NVH Medicinal**
  **21000 Dijon (FR)**
- **Centre Hospitalier Universitaire De Dijon**
  **21000 Dijon (FR)**

(72) Inventeurs:
- **VANDROUX, David**
  **F-21000 Dijon (FR)**
- **DE MAISTRE, Emmanuel**
  **F-21121 Fontaine-les-Dijon (FR)**
- **DUMONT DI LEONE, Laure**
  **21000 Dijon (FR)**
- **COUTARD, François**
  **F-30100 Ales (FR)**

(74) Mandataire: **Regimbeau**
**139, rue Vendôme**
**69477 Lyon Cedex 06 (FR)**

(56) Documents cités:
- **FAVALORO EMMANUEL J: "Evaluation of commercial von Willebrand factor collagen binding assays to assist the discrimination of types 1 and 2 von Willebrand disease", THROMBOSIS AND HAEMOSTASIS, vol. 104, no. 5, Sp. Iss. SI, novembre 2010 (2010-11), pages 1009-1021, XP009167719, ISSN: 0340-6245**
- **BARONCIANI L ET AL: "von Willebrand factor collagen binding assay in von Willebrand disease type 2A, 2B, and 2M.", JOURNAL OF THROMBOSIS AND HAEMOSTASIS : JTH SEP 2006, vol. 4, no. 9, septembre 2006 (2006-09), pages 2088-2090, XP002693778, ISSN: 1538-7933**
- **HEEMSKERK J W M ET AL: "Collagen surfaces to measure thrombus formation under flow: possibilities for standardization.", JOURNAL OF THROMBOSIS AND HAEMOSTASIS : JTH APR 2011, vol. 9, no. 4, avril 2011 (2011-04), pages 856-858, XP002693779, ISSN: 1538-7836**
- **PUGH NICHOLAS ET AL: "Synergism between platelet collagen receptors defined using receptor-specific collagen-mimetic peptide substrata in flowing blood", BLOOD, vol. 115, no. 24, juin 2010 (2010-06), pages 5069-5079, XP002693780,**

- LISMAN TON ET AL: "A single high-affinity binding site for von Willebrand factor in collagen III, identified using synthetic triple-helical peptides", BLOOD, AMERICAN SOCIETY OF HEMATOLOGY, US, vol. 108, no. 12, 1 décembre 2006 (2006-12-01), pages 3753-3756, XP002425786, ISSN: 0006-4971, DOI: 10.1182/BLOOD-2006-03-011965
- HERR ANDREW B ET AL: "Structural Insights into the Interactions between Platelet Receptors and Fibrillar Collagen", JOURNAL OF BIOLOGICAL CHEMISTRY, vol. 284, no. 30, juillet 2009 (2009-07), pages 19781-19785, XP002693781, ISSN: 0021-9258
- BRONDIJK T HARMA C ET AL: "Implications for collagen I chain registry from the structure of the collagen von Willebrand factor A3 domain complex", PROCEEDINGS OF THE NATIONAL ACADEMY OF SCIENCES OF THE UNITED STATES OF AMERICA, vol. 109, no. 14, avril 2012 (2012-04), pages 5253-5258, XP002693782, ISSN: 0027-8424
- WERKMEISTER J A ET AL: "Recombinant protein scaffolds for tissue engineering", BIOMEDICAL MATERIALS IOP PUBLISHING LTD. UK, vol. 7, no. 1, février 2012 (2012-02), XP002693783, ISSN: 1748-605X

SEQUENCE LISTING

<110> NVH Medicinal

<120> Protéines recombinantes dérivées du collagène à activité de liaison au facteur Willebrand

<130> 364053D29804

<150> FR 1259996
<151> 19 octobre 2012

<160> 8

<170> PatentIn version 3.5

<210> 1
<211> 552
<212> DNA
<213> Artificial Sequence

<220>
<223> Séquence dérivée du collagène

<220>
<221> CDS
<222> (1)..(552)

<220>
<221> sig_peptide
<222> (1)..(69)

<400> 1

```
atg atg agc ttc gtg cag aag ggg agc tgg ctg ctt ctc gct ctg ctt      48
Met Met Ser Phe Val Gln Lys Gly Ser Trp Leu Leu Leu Ala Leu Leu
1               5                   10                  15

cat cct act att atc ctg gca cag ggg cgc ccc gga gct cct gga gag      96
His Pro Thr Ile Ile Leu Ala Gln Gly Arg Pro Gly Ala Pro Gly Glu
            20                  25                  30

aga gga ttg cct gga cct cca ggg ccc aga gga gct gct gga gaa cct     144
Arg Gly Leu Pro Gly Pro Pro Gly Pro Arg Gly Ala Ala Gly Glu Pro
            35                  40                  45

ggc aga gat ggc gtc cct gga gga cca gga atg agg ggc atg ccc gga     192
Gly Arg Asp Gly Val Pro Gly Gly Pro Gly Met Arg Gly Met Pro Gly
    50                  55                  60

agc cca gga gga cca gga agc gat ggg aag cca ggg cct ccc gga agc     240
Ser Pro Gly Gly Pro Gly Ser Asp Gly Lys Pro Gly Pro Pro Gly Ser
65                  70                  75                  80

cag gga gaa agc ggc aga cca gga cct cct gga gag aac gga ttc cct     288
Gln Gly Glu Ser Gly Arg Pro Gly Pro Pro Gly Glu Asn Gly Phe Pro
                85                  90                  95

gga gaa agg ggg gat gca ggc gct cct ggg gca cca gga cct cgt ggc     336
Gly Glu Arg Gly Asp Ala Gly Ala Pro Gly Ala Pro Gly Pro Arg Gly
            100                 105                 110

cag ccc ggg gtg atg gga ttc ccc ggg gat gcc ggg gca cct ggt gca     384

Gln Pro Gly Val Met Gly Phe Pro Gly Asp Ala Gly Ala Pro Gly Ala
            115                 120                 125

cct ggc cca cgt ggg cag cct gga gtc atg ggg ttc cct gga cca cct     432
Pro Gly Pro Arg Gly Gln Pro Gly Val Met Gly Phe Pro Gly Pro Pro
            130                 135                 140

ggc cca cca ggc cct ccc gga cca cct gga cct cca ggc cct cct ggc     480
Gly Pro Pro Gly Pro Pro Gly Pro Pro Gly Pro Pro Gly Pro Pro Gly
145                 150                 155                 160

cct cct gga cct cct gga cca cct gga cct cca ggc cca aga ggc gac     528
Pro Pro Gly Pro Pro Gly Pro Pro Gly Pro Pro Gly Pro Arg Gly Asp
                165                 170                 175

aag gga cct cct gga cct gga tct                                      552
Lys Gly Pro Pro Gly Pro Gly Ser
                180
```

<210> 2
<211> 184
<212> PRT
<213> Artificial Sequence

<220>
<223> Séquence dérivée du collagène

<400> 2

```
Met Met Ser Phe Val Gln Lys Gly Ser Trp Leu Leu Leu Ala Leu Leu
1               5                   10                  15

His Pro Thr Ile Ile Leu Ala Gln Gly Arg Pro Gly Ala Pro Gly Glu
            20              25                  30

Arg Gly Leu Pro Gly Pro Pro Gly Pro Arg Gly Ala Ala Gly Glu Pro
        35              40              45

Gly Arg Asp Gly Val Pro Gly Gly Pro Gly Met Arg Gly Met Pro Gly
    50              55              60

Ser Pro Gly Gly Pro Gly Ser Asp Gly Lys Pro Gly Pro Pro Gly Ser
65              70              75              80

Gln Gly Glu Ser Gly Arg Pro Gly Pro Pro Gly Glu Asn Gly Phe Pro
            85              90                  95

Gly Glu Arg Gly Asp Ala Gly Ala Pro Gly Ala Pro Gly Pro Arg Gly
        100             105             110

Gln Pro Gly Val Met Gly Phe Pro Gly Asp Ala Gly Ala Pro Gly Ala
        115             120             125

Pro Gly Pro Arg Gly Gln Pro Gly Val Met Gly Phe Pro Gly Pro Pro

        130             135             140

Gly Pro Pro Gly Pro Pro Gly Pro Pro Gly Pro Pro Gly Pro Pro Gly
145             150             155             160

Pro Pro Gly Pro Pro Gly Pro Pro Gly Pro Pro Gly Pro Arg Gly Asp
            165             170             175

Lys Gly Pro Pro Gly Pro Gly Ser
            180
```

<210> 3
<211> 163
<212> PRT
<213> Artificial Sequence

<220>
<223> Séquence dérivée du collagène

<220>
<221> SIGNAL
<222> (1)..(23)

<400> 3

```
        Met Met Ser Phe Val Gln Lys Gly Ser Trp Leu Leu Leu Ala Leu Leu
        1               5                   10                  15

        His Pro Thr Ile Ile Leu Ala Gln Gly Arg Pro Gly Ala Pro Gly Glu
                    20                  25                  30

        Arg Gly Leu Pro Gly Pro Pro Gly Pro Arg Gly Ala Ala Gly Glu Pro
                35                  40                  45

        Gly Arg Asp Gly Val Pro Gly Gly Pro Gly Met Arg Gly Met Pro Gly
                50                  55                  60

        Ser Pro Gly Gly Pro Gly Ser Asp Gly Lys Pro Gly Pro Pro Gly Ser
        65                  70                  75                  80

        Gln Gly Glu Ser Gly Arg Pro Gly Pro Pro Gly Glu Asn Gly Phe Pro
                        85                  90                  95

        Gly Glu Arg Gly Asp Ala Gly Ala Pro Gly Ala Pro Gly Pro Arg Gly
                    100                 105                 110

        Gln Pro Gly Val Met Gly Phe Pro Gly Pro Pro Gly Pro Pro Gly Pro
                    115                 120                 125

        Pro Gly Pro Pro Gly Pro Pro Gly Pro Pro Gly Pro Pro Gly Pro Pro

                    130                 135                 140

        Gly Pro Pro Gly Pro Pro Gly Pro Arg Gly Asp Lys Gly Pro Pro Gly
        145                 150                 155                 160
```

Pro Gly Ser

<210> 4
<211> 205
<212> PRT
<213> Artificial Sequence

<220>
<223> Séquence dérivée du collagène

<220>
<221> SIGNAL
<222> (1)..(23)

<400> 4

```
Met Met Ser Phe Val Gln Lys Gly Ser Trp Leu Leu Leu Ala Leu Leu
1               5               10              15

His Pro Thr Ile Ile Leu Ala Gln Gly Arg Pro Gly Ala Pro Gly Glu
            20              25              30

Arg Gly Leu Pro Gly Pro Pro Gly Pro Arg Gly Ala Ala Gly Glu Pro
        35              40              45

Gly Arg Asp Gly Val Pro Gly Gly Pro Gly Met Arg Gly Met Pro Gly
    50              55              60

Ser Pro Gly Gly Pro Gly Ser Asp Gly Lys Pro Gly Pro Pro Gly Ser
65              70              75              80

Gln Gly Glu Ser Gly Arg Pro Gly Pro Pro Gly Glu Asn Gly Phe Pro
            85              90              95

Gly Glu Arg Gly Asp Ala Gly Ala Pro Gly Ala Pro Gly Pro Arg Gly
        100             105             110

Gln Pro Gly Val Met Gly Phe Pro Gly Asp Ala Gly Ala Pro Gly Ala
        115             120             125

Pro Gly Pro Arg Gly Gln Pro Gly Val Met Gly Phe Pro Gly Asp Ala
        130             135             140

Gly Ala Pro Gly Ala Pro Gly Pro Arg Gly Gln Pro Gly Val Met Gly
145             150             155             160

Phe Pro Gly Pro Pro Gly Pro Pro Gly Pro Pro Gly Pro Pro Gly Pro
            165             170             175

Pro Gly Pro Pro Gly Pro Pro Gly Pro Pro Gly Pro Pro Gly Pro Pro
        180             185             190

Gly Pro Arg Gly Asp Lys Gly Pro Pro Gly Pro Gly Ser
        195             200             205
```

<210> 5
<211> 226
<212> PRT
<213> Artificial Sequence

<220>
<223> Séquence dérivée du collagène

<220>
<221> SIGNAL
<222> (1)..(23)

<400> 5

```
Met Met Ser Phe Val Gln Lys Gly Ser Trp Leu Leu Leu Ala Leu Leu
1               5               10              15

His Pro Thr Ile Ile Leu Ala Gln Gly Arg Pro Gly Ala Pro Gly Glu
            20              25              30

Arg Gly Leu Pro Gly Pro Pro Gly Pro Arg Gly Ala Ala Gly Glu Pro
            35              40              45

Gly Arg Asp Gly Val Pro Gly Gly Pro Gly Met Arg Gly Met Pro Gly
    50              55              60

Ser Pro Gly Gly Pro Gly Ser Asp Gly Lys Pro Gly Pro Pro Gly Ser
65              70              75              80

Gln Gly Glu Ser Gly Arg Pro Gly Pro Pro Gly Glu Asn Gly Phe Pro
            85              90              95

Gly Glu Arg Gly Asp Ala Gly Ala Pro Gly Ala Pro Gly Pro Arg Gly
            100             105             110

Gln Pro Gly Val Met Gly Phe Pro Gly Asp Ala Gly Ala Pro Gly Ala
            115             120             125

Pro Gly Pro Arg Gly Gln Pro Gly Val Met Gly Phe Pro Gly Asp Ala
```

130     135     140

```
Gly Ala Pro Gly Ala Pro Gly Pro Arg Gly Gln Pro Gly Val Met Gly
145                 150                 155                 160

Phe Pro Gly Asp Ala Gly Ala Pro Gly Ala Pro Gly Pro Arg Gly Gln
                165                 170                 175

Pro Gly Val Met Gly Phe Pro Gly Pro Pro Gly Pro Pro Gly Pro Pro
            180                 185                 190

Gly Pro Pro Gly Pro Pro Gly Pro Pro Gly Pro Pro Gly Pro Pro Gly
        195                 200                 205

Pro Pro Gly Pro Pro Gly Pro Arg Gly Asp Lys Gly Pro Pro Gly Pro
    210                 215                 220

Gly Ser
225
```

<210> 6
<211> 12
<212> PRT
<213> Artificial Sequence

<220>
<223> Motif liaison facteur Willebrand

<400> 6

```
Gly Pro Arg Gly Gln Pro Gly Val Met Gly Phe Pro
1               5                   10
```

<210> 7
<211> 9
<212> PRT
<213> Artificial Sequence

<220>
<223> Linker

<400> 7

```
Gly Asp Ala Gly Ala Pro Gly Ala Pro
1               5
```

<210> 8
<211> 42
<212> PRT
<213> Artificial Sequence

<220>
<223> (Linker et motif de liaison facteur Willebrand)x2

<400> 8

```
Gly Asp Ala Gly Ala Pro Gly Ala Pro Gly Pro Arg Gly Gln Pro Gly
1               5               10              15

Val Met Gly Phe Pro Gly Asp Ala Gly Ala Pro Gly Ala Pro Gly Pro
        20              25              30

Arg Gly Gln Pro Gly Val Met Gly Phe Pro
        35              40
```

## Description

L'invention se rapporte à des protéines recombinantes comprenant au moins deux motifs dérivés du collagène permettant à ces protéines de se lier spécifiquement au facteur vonWillebrand (vWF). Ces protéines peuvent être mises en oeuvre dans différents tests biologiques *in vitro* afin de mesurer la capacité du facteur Willebrand à se lier au collagène.

Le facteur Willebrand est une glycoprotéine adhésive formée de multimères de haut poids moléculaire produite par les cellules endothéliales et les mégacaryocytes. Il est stocké dans les plaquettes et présent sous forme circulante dans le plasma. La protéine mature de Willebrand est formée de monomères de 250 kDa regroupés en multimères par des ponts disulfures qui peuvent atteindre une masse de l'ordre de 20 MDaet représenter jusqu'à une centaine de monomères. La multimérisation est un processus majeur car si tous les multimères sont capables de lier le facteur VIII,quelque soit leur taille, les multimères de haut poids moléculaire sont les formes les plus actives pour assurer l'adhésion plaquettaire. La formation de ces multimères est liée à la présence dans la séquence du monomère de domaines dits « structuraux », à savoir un domaine riche en cystéine (Cysteinknotdomain) et un domaine D3 permettant une multimérisation optimale des dimères de Willebrand (Springer TA, J ThrombHaemost. 2011 Jul; 9 Suppl 1:130-43). Il existe plusieurs domaines D dans la séquence du monomère qui se distinguent par la présence de séquences consensus permettant la liaison des isomérases disulfides. A côté de ces domaines « structuraux » sont présents des domaines dits « fonctionnels » qui incluent trois domaines : A1, A2 et A3 avec les domaines A1 et A3 contenant un site de liaison au collagène, le domaine A1 un site de liaison aux récepteurs plaquettaires GpIb et αIIβIII et le domaine A2, des sites de clivage protéolytiques (Schneppenheim R, Thromb Res.2011;128 Suppl 1:S3-7).

La formation des multimères de Willebrand est régulée par la protéine ADAMTS13 (A Disintegrinand Metalloprotease with Thrombospondin Motifs) par coupure protéolytique au niveau du domaine A2. Un déficit en ADAMTS 13 conduit aupurpura thrombotique thrombocytopénique (PTT) consécutifà l'adhésion des plaquettes à des multimères de facteur Willebrand de très grande taille à l'origine de thrombiplaquettairedans la microcirculation et d'une hémolyse.

Le facteur Willebrand joue deux rôles majeurs en permettant 1) l'adhésion des plaquettes consécutivement à une lésion de l'endothélium et la mise à nu du collagène, cette adhésion devenant très dépendante du facteur Willebrand en cas de vitesses de flux ou forces de cisaillement élevées et 2) le transport et la protection du facteur VIII de la coagulation dans le sang circulant.Au niveau plasmatique, sa concentration est de l'ordre de 10 μg/mL et les valeurs plasmatiques de ce facteur sont situées entre 50 et 150 UI/dL en activité.

La maladie de Willebrand est la maladie hémorragique la plus fréquente avec une prévalence de l'ordre de 1% dans la population générale. Elle se caractérise par des saignements principalement au niveau des muqueuses. Il existe différents types et sous-types de la maladie de Willebrand caractérisés par des défauts quantitatifs et qualitatifs de ce facteur. C'est ainsi que l'on distingue:

- le type 1, correspondant à un déficit quantitatif partiel en facteur Willebrand. Il représente 70 à 80 % des patients atteints,
- le type 3, qui correspond à un déficit quantitatif total de ce facteur, associé à des hémorragies sévères et à un effondrement marqué du facteur VIII,
- le type 2 correspondant à un déficit qualitatif en Willebrand. Il est divisé en plusieurs sous-types, à savoir 1) le sous-type 2A qui présente une diminution de l'affinité pour les plaquettes liée à l'absence de multimères de haut poids moléculaire, 2) le sous-type 2B caractérisé par une augmentation de l'affinité pour le récepteur plaquettaire GpIb qui se traduit par une élimination ou une perte des multimères de haut poids moléculaire, 3) le sous-type 2N qui possèdeune diminution de l'affinité pour le Facteur VIII se traduisant par un déficit en facteur VIII avec un taux de Willebrand normal et 4) le sous-type 2M qui regroupe tous les autres types de déficit de la fonction du facteur Willebrand qui ne sont pas liés à une perte des multimères de haut poids moléculaire.

Le facteur Willebrand joue un rôle majeur dans l'interaction indirecte entre le récepteur plaquettaire GpIb et le collagène. Les formes multimériques de haut poids moléculaire permettent le recrutement des plaquettes au niveau des sites lésés des territoires vasculaires soumis à des vitesses de flux élevées. Le facteur Willebrand se fixe au collagène par l'intermédiaire de ses domaines A3 et A1 tandis que le domaine A1 lie en plus le récepteur GpIb plaquettaire.

Les collagènes sont les constituants structuraux principaux de la matrice extracellulaire de tous les organismes multicellulaires. Il s'agit d'une famille de protéines composée de 28 types différents jouant un rôle au cours du développement et dans l'homéostasie tissulaire. Ils sont capables de s'assembler en différentes structures supramoléculaires sous forme de fibrilles, de microfibrilles ou encore de réseaux. Les collagènes présentent la caractéristique commune de contenir un ou plusieurs domaine(s) ayant une structure en triple hélice formée de trois chaînes polypeptidiques, ou chaînesα, enroulées les unes autour des autres. Cette caractéristique est permise par la présence, tous les trois acides aminés, d'une glycine au niveau des motifs hélicoïdaux qui sont constitués de séquences répétées de type G-X-Y où X est souvent une proline et Y une hydroxyproline (Chen C et Raghunath M. Fibrogenesis Tissue Repair. 2009 Dec 15;2:7).

Les collagènes de type II et III possèdent un site unique, de haute affinité, pour le domaine A3 du facteur Willebrand (Herr AB et Farndale RW, J BiolChem. 2009 Jul 24; 284(30):19781-5*)*. Ce motif peptidique est localisé entre les acides aminés 403 à 413 et se compose de GPRGQOGVMGFO avec certains acides aminés critiques pour la fixation au facteur Willebrand (Lisman T et coll., Blood. 2006 Dec 1; 108(12) : 3753-6). Cette séquence peptidique est également impliquée dans l'interaction entre le collagène de type II, III, IV et les récepteurs DDR1 et DDR2 (Discoidin Domain Receptor) (*Xu H et coll., Matrix Biol. 2011 Jan ; 30*(*1*) *: 16-26),* exprimés par les cellules épithéliales et les cellules d'origine mésenchymateuse respectivement, ainsi qu'avec la protéine SPARC (SecretedProteinAcidic and Rich in Cysteine, également nommée ostéonectine), impliquée dans les interactions cellule-matrice extracellulaire (Giudici C et coll., J BiolChem. 2008 Jul11;283(28):19551-60). Plus récemment, Brondijk et coll. ont décrit les motifs de reconnaissance possiblement impliqués dans la liaison du collagène de type I au facteur Willebrand, à savoir le motif RGQAGVMF pour la chaîne alpha 1 et RGEOGNIGF pour la chaîne alpha 2 qui sont des motifs dits « dégénérés » par rapport au motif RGQOGVMGF présent dans la séquence du collagène de type III (Brondijk TH et coll. Proc NatlAcadSci U S A. 2012 Apr 3;109(14):5253-8).Par ailleurs, Verkleij et coll. avaient identifié comme potentiel site de liaison, les acides aminés 541 à 558 composés de GAAGPOGPOGSAGTOGLQ (Verkleij et coll., Blood. 1998 May 15; 91 (10) : 3808-16). Ce motif peptidique est localisé entre le motif Willebrand décrit par Lisman et coll. et le motif de liaison à l'intégrine $\alpha 1\beta 2$ (motif GMOGER). Ce motif n'a pas été repris dans d'autres travaux par la suite.

A noter que Bonnefoy et coll. ont démontré que le domaine A1 du facteur Willebrand pouvait se substituer au domaine A3 pour permettre la fixation des plaquettes à du collagène pour des vitesses de flux élevées sans qu'ils aient pu depuis préciser si cette interaction s'effectuait au niveau du même site de liaison au collagène que pour le domaine A3 *(*Bonnefoy A et coll. J ThrombHaemost. 2006 Oct;4(10):2151-61).

La classification des patients atteints de la maladie de Willebrand nécessite l'utilisation de différents tests de laboratoire. Le dosage de l'antigène du facteur Willebrand (VWF:Ag) est souvent réalisé en première ligne en cas de suspicion mais il ne permet pas de quantifier l'activité du facteur Willebrand. Le recours à des tests dits fonctionnels, mesurant l'activité du facteur Willebrand est indispensable pour détecter des anomalies fonctionnelles du facteur Willebrand permettant la classification des patients dans les différents sous-types, notamment lorsque ces anomalies ne sont pas associées à une faible concentration en facteur Willebrand. Comme précisé précédemment, le facteur Willebrand permet l'adhésion des plaquettes au collagène en faisant le lien entre le collagène et le récepteur plaquettaire GpIb. Les tests fonctionnels utilisés à ce jour sont surtout ciblés sur la liaison Willebrand/plaquettes.

La mesure de la liaison du facteur Willebrand au collagène n'est pas utilisée en routine et ce, principalement du fait que les tests actuels ne sont pas adaptés à l'urgence et au cas par cas. Cependant, la mesure de la liaison du facteur Willebrand au collagène ou VWF:CB (vonWillebrandFactor:CollagenBinding) possède une meilleure sensibilité pour détecter la présence de Willebrand de haut poids moléculaire (Favaloro EJ, Thromb Haemost.2010 Nov;104(5):1009-21).

Afin de faciliter la classification des patients en fonction des sous-types, on calcule le rapport (ratio) entre l'activité liaison du Willebrand aux plaquettes ou du collagène au Willebrand sur la concentration de l'antigène Willebrand (vWF:Ag). Un rapport activité/Ag égal ou supérieur à 0,7 est obtenu pour les patients normaux et de type I et un rapport inférieur à 0,7 est caractéristique des sous-types 2A, 2B et 2M. Les sous-types 2N ne peuvent pas être détectés par cette mesure.

Les tests ELISA basés sur la mesure de la liaison du Willebrand au collagène permettent une bonne discrimination entre les patients de type 1 et de type 2A/2B (Favaloro EJ, Thromb Haemost.2010 Nov;104(5):1009-21*)* et entre les types 2A et 2M (Baronciani L et coll. J Thromb Haemost.2006 Sep;4(9):2088-90*)*.

Bien que ces résultats semblent encourageants, un débat existe quant à l'intérêt de ce test pour la classification des patients. Ce débat porte principalement sur la caractérisation de l'absence ou non des multimères de haut poids moléculaire qui est un enjeu important, aussi bien pour l'aide à la classification des patients Willebrand que pour ceux ayant un déficit acquis en multimères de haut poids moléculaire. Il existe à ce jour pas moins de 7 trousses ELISA commercialisées sans compter celles qui sont développées en interne par certains laboratoires. Ces trousses utilisent des préparations de collagène d'origine et de nature différentes, ce qui complique la comparaison des résultats entre les différentes études. A cela s'ajoute le manque d'homogénéité entre les lots produits. Les collagènes classiquement utilisés sont le collagène de type I, le type III, le type IV et le type VI. Ces collagènes d'origine animale ne sont pas purs à 100 % et peuvent être à l'origine des résultats discordants décrits. De là, un besoin important d'uniformatisation des préparations de collagène utilisées, aussi bien pour les tests ELISA que pour les tests mesurant la liaison du Willebrand au collagène en conditions de flux. Ce point a été largement détaillé et discuté dans une directive du sous-comité Biorhéologie de l'ISTH tendant vers une standardisation des tests utilisant le collagène (Heemskerk JWM et coll. J Thromb Haemost.2011 Apr;9(4):856-8*)*.

L'ingénierie de protéines dérivées de la matrice extracellulaire permet aujourd'hui de disposer de protéines possédant des propriétés équivalentes aux protéines natives (Werkmeister J et Ramshaw J. Biomed Mater. 2012 Feb;7(1):012002). Utilisant les technologies de l'ADN recombinant, il est possible d'obtenir, de façon reproductible, des lots stables de ces protéines en très grande quantité. Bien que les collagènes natifs aient déjà été produits en cellules eucaryotes avec succès (brevet EP 2383338), la production de protéines recombinantes dérivées du collagène issues d'un procédé d'ingénierie est une approche récente qui n'a pas été à ce jour utilisée pour produire des protéines dérivées du collagène capables de se lier au facteur Willebrand et d'être utilisées dans un test d'activité de ce facteur, telles que les polypeptides de la présente invention.

Contrairement au test d'activité de liaison du Willebrand au récepteur plaquettaire GpIb qui existe sous forme automatisé, il n'existe pas à ce jour de test automatisé pour la mesure de la liaison du Willebrand au collagène. Les tests automatisés sont généralement basés sur le principe de l'agglutination de particules recouvertes d'un réactif. Le test d'agglutination le plus utilisé en diagnostic utilise des billes de latex qui, pour l'heure, n'ont pas pu être recouvertes de collagène pour leur utilisation comme réactif. Les propriétés physico-chimiques des collagènes utilisés classiquement et, notamment, le fait d'être soluble uniquement à pH acide, compliquent l'utilisation de ce type de billes. A l'inverse, les polypeptides selon l'invention sont solubles à pH physiologique, permettant une adsorption optimale en surface de particules. Par ailleurs, leur masse est plus petite que celle des collagènes natifs ce qui devrait permettre une meilleure adsorption sur les particules. Enfin, l'ajout possible par ingénierie de cystéines dans la séquence du polypeptide permet de réaliser un greffage ciblé entre une particule fonctionnalisée avec une fonction de type maléimide en surface de la particule et la cystéine présente dans la protéine. Les avantages spécifiques des polypeptides selon l'invention couplés à leur activité permettent le développement du premier test automatisé de la mesure de la liaison du Willebrand plasmatique au collagène.

Résumé de l'invention

La présente invention a pour objet un procédé pour déterminer l'activité de liaison du facteur Willebrand au collagène dans un échantillon biologique comprenant les étapes suivantes :

a) Mise à disposition d'un polypeptide choisi parmi :

- le polypeptide comprenant la séquence de la position 24 à la position 184 de la SEQ ID No.2,
- le polypeptide comprenant la séquence de la position 24 à la position 205 de la SEQ ID No.4,
- le polypeptide comprenant la séquence de la position 24 à la position 226 de la SEQ ID No. 5,
- un polypeptidese liant au facteur Willebrand et comprenant le motif peptidique $Z_1$-GPRGQPGVMGFP-$Z_2$-GPR-GQPGVMGFP et le motif peptidique $(GPP)_k$

dans lesquels indépendamment
$Z_1$ représente un linker comprenant de 6 à 12 acides aminés,
$Z_2$ représente un linker comprenant de 6 à 12 acides aminés,
k est un nombre entier compris entre 4 et 15 ;
b) Mise en contact de l'échantillon biologique avec ledit polypeptide ;
c) Mesure de la liaison du facteur Willebrand de l'échantillon biologique au polypeptide de l'étape a) pour mesurer l'activité de liaison du facteur Willebrand au collagène.

Les polypeptides décrits ci-dessus présentent une activité et une affinité de liaison au facteur Willebrand équivalente à celle des collagènes type I et III. Ces polypeptides présentent des caractéristiques physico-chimiques avantageuses, notamment le fait qu'ils soient stables à un pH physiologique, et également qu'ils soient capables de lier le facteur Willebrand sous forme non fibrillaire.

De préférence, $Z_1$ et $Z_2$ représentent indépendamment un motif peptidique de formule $(GAA_1AA_2)_{n1}(GAA_3AA_4)_{n2}(GAA_5AA_6)_{n3}(GAA_7AA_8)_{n4}$ dans lequel indépendamment :

- $AA_1$, $AA_3$, $AA_5$ et $AA_7$ représentent indépendamment les acides aminés D ou A,
- $AA_2$, $AA_4$, $AA_6$ et $AA_8$ représentent indépendamment les acides aminés A ou P,
- $n_1$, $n_2$, $n_3$ et $n_4$ sont choisis indépendamment parmi 0, 1, 2, 3 ou 4 et la somme $n_1+n_2+n_3+n_4$ est égale à 2, 3 ou 4.

Plus préférentiellement, $Z_1$ et $Z_2$ représentent le motif peptidique GDAGAPGAP de la SEQ ID No.7.

Un deuxième objet de la présente invention est un procédé de diagnostic de la maladie de Willebrand chez un patient comprenant les étapes suivantes :

a) Mise à disposition d'un polypeptide choisi parmi :

- le polypeptide comprenant la séquence de la position 24 à la position 184 de la SEQ ID No.2,

- le polypeptide comprenant la séquence de la position 24 à la position 205 de la SEQ ID No.4,
- le polypeptide comprenant la séquence de la position 24 à la position 226 de la SEQ ID No. 5,
- un polypeptide se liant au facteur Willebrand et comprenant le motif peptidique $Z_1$-GPRGQPGVMGFP-$Z_2$-GPR-GQPGVMGFP et le motif peptidique $(GPP)_k$

dans lesquels indépendamment
$Z_1$ représente un linker comprenant de 6 à 12 acides aminés,
$Z_2$ représente un linker comprenant de 6 à 12 acides aminés,
k est un nombre entier compris entre 4 et 15 ;
b) Mise en contact d'un échantillon biologique préalablement prélevé chez le patient avec ledit polypeptide ;
c) Mesure de la liaison du facteur Willebrand présent dans l'échantillon biologique au polypeptide de l'étape a) pour mesurer l'activité de liaison du facteur Willebrand au collagène.

De façon avantageuse, le procédé de diagnostic est mis en oeuvre pour diagnostiquer les types 1, 2, 2A, 2B, 2M et 3 de la maladie de Willebrand.

Préférentiellement, $Z_1$ et $Z_2$ représentent indépendamment un motif peptidique de formule $(GAA_1AA_2)_{n1}(GAA_3AA_4)_{n2}(GAA_5AA_6)_{n3}(GAA_7AA_8)_{n4}$
dans lequel indépendamment
$AA_1$, $AA_3$, $AA_5$ et $AA_7$ représentent indépendamment les acides aminés D ou A,
$AA_2$, $AA_4$, $AA_6$ et $AA_8$ représentent indépendamment les acides aminés A ou P,
$n_1$, $n_2$, $n_3$ et $n_4$ sont choisis indépendamment parmi 0, 1, 2, 3 ou 4 et la somme $n_1+n_2+n_3+n_4$ est égale à 2, 3 ou 4.

Plus préférentiellement, $Z_1$ et $Z_2$ représentent le motif peptidique GDAGAPGAP de la SEQ ID No.7.

Dans des modes de réalisation avantageux de l'invention, les procédés de diagnostic de la maladie de Willebrand comprennent en outre la détermination de la quantité de facteur Willebrand présent dans l'échantillon biologique.

De préférence, le polypeptide de l'étape a) est fixé sur un support solide.

De préférence, les étapes b) et c) sont réalisées en conditions de flux.

L'invention a également pour objet un polypeptide choisi parmi :

- le polypeptide comprenant la séquence de la SEQ ID No.2,
- le polypeptide comprenant la séquence de la position 24 à la position 184 de la SEQ ID No.2,
- le polypeptide comprenant la séquence de la SEQ ID No.4,
- le polypeptide comprenant la séquence de la position 24 à la position 205 de la SEQ ID No.4,
- le polypeptide comprenant la séquence de la SEQ ID No. 5,
- le polypeptide comprenant la séquence de la position 24 à la position 226 de la SEQ ID No. 5,
- un polypeptide se liant au facteur Willebrand et comprenant le motif peptidique $Z_1$-GPRGQPGVMGFP-$Z_2$-GPR-GQPGVMGFP et le motif peptidique $(GPP)_k$

dans lesquels indépendamment
$Z_1$ représente un linker comprenant de 6 à 12 acides aminés,
$Z_2$ représente un linker comprenant de 6 à 12 acides aminés,
k est un nombre entier compris entre 4 et 15.

Dans des modes de réalisation préférés, les polypeptides selon la présente invention sont fixés sur un support solide.

L'invention se rapporte aussi à des kits pour déterminer l'activité de liaison du facteur Willebrand au collagène comprenant un polypeptide choisi parmi :

- le polypeptide comprenant la séquence de la position 24 à la position 184 de la SEQ ID No.2,
- le polypeptide comprenant la séquence de la position 24 à la position 205 de la SEQ ID No.4,
- le polypeptide comprenant la séquence de la position 24 à la position 226 de la SEQ ID No. 5,
- un polypeptide d'au moins 100 acides aminés se liant au facteur Willebrand et comprenant le motif peptidique $Z_1$-GPRGQPGVMGFP-$Z_2$- GPRGQPGVMGFP et le motif peptidique $(GPP)_k$

dans lesquels indépendamment
$Z_1$ représente un linker comprenant de 6 à 12 acides aminés,
$Z_2$ représente un linker comprenant de 6 à 12 acides aminés,
k est un nombre entier compris entre 4 et 15 ;
un réactif de détection pour déterminer la liaison du facteur Willebrand au collagène.

Avantageusement, les kits selon la présente invention comprennent en outre un anticorps se liant au facteur Willebrand.

De préférence, le polypeptide est fixé sur un support solide.

Listage de séquences

SEQ IDNo. 1 : Polynucléotide codant pour un polypeptide comprenant deux motifs de liaison au facteur Willebrand
SEQ ID No. 2 : Polypeptide comprenant deux motifs de liaison au facteur Willebrand
SEQ ID No. 3 : Polypeptide comprenant un motif de liaison au facteur Willebrand
SEQ ID No. 4 : Polypeptide comprenant trois motifs de liaison au facteur Willebrand
SEQ ID No. 5 : Polypeptide comprenant quatre motifs de liaison au facteur Willebrand
SEQ ID No. 6 : Motif de liaison au facteur Willebrand
SEQ ID No. 7 : Linker
SEQ ID No. 8 : Linker-motif de liaison au facteur Willebrand-Linker-motif de liaison au facteur Willebrand

Description détaillée de l'invention

Le facteur Willebrand se lie au collagène et des altérations de l'activité de liaison de ce facteur au collagène sont à l'origine des différents types de la maladie de Willebrand. Le collagène est une protéine difficilement produite ou purifiée et dont la manipulation n'est pas aisée. En effet, de par sa structure, le collagène adhère aux surfaces et il n'est pas soluble dans l'eau. La présente invention propose des polypeptides recombinants capables de remplacer le collagène dans tous les essais et kits de mesure de la liaison du facteur Willebrand au collagène. Les polypeptides de la présente invention peuvent être produits dans différents systèmes cellulaires dont les cellules CHO.Ils sont facilement purifiés et forment des trimères en solution dont la structure est similaire à celle du collagène. Ces polypeptides sont par ailleurs solubles et notamment à pH physiologique ce qui permet de les fixer aisément sur des supports solides tels que les billes de latex par exemple.

De façon remarquable, les données expérimentales rapportées ci-dessous montrent que les polypeptides selon l'invention présentent une activité et une affinité de liaison au facteur Willebrand égale à celle du collagène III humain. Les polypeptides selon la présente invention trouvent donc de nombreuses applications dans les procédés, tests et kits basés sur la détermination/mesure de la liaison du facteur Willebrand au collagène.

L'invention se rapporte en premier lieu à des polypeptides se liant au facteur Willebrand choisis parmi :

- le polypeptide de la SEQ ID No.2,
- le polypeptide de la position 24 à la position 184 de la SEQ ID No.2,
- le polypeptide de la SEQ ID No.4,
- le polypeptide de la position 24 à la position 205 de la SEQ ID No.4,
- le polypeptide de la SEQ ID No. 5,
- le polypeptide de la position 24 à la position 226 de la SEQ ID No. 5,
- un polypeptide se liant au facteur Willebrand et comprenant le motif peptidique $Z_1$-GPRGQPGVMGFP-$Z_2$- GPR-GQPGVMGFP et le motif peptidique $(GPP)_k$

dans lesquels indépendamment
$Z_1$ représente un linker comprenant de 6 à 12 acides aminés,
$Z_2$ représente un linker comprenant de 6 à 12 acides aminés,
k est un nombre entier compris entre 4 et 15.

L'invention se rapporte également à des polypeptides se liant au facteur Willebrand choisis parmi :

- le polypeptide comprenant la séquence de la SEQ ID No.2,
- le polypeptide comprenant la séquence de la position 24 à la position 184 de la SEQ ID No.2,
- le polypeptide comprenant la séquence de la SEQ ID No.4,
- le polypeptide comprenant la séquence de la position 24 à la position 205 de la SEQ ID No.4,
- le polypeptide comprenant la séquence de la SEQ ID No. 5,
- le polypeptide comprenant la séquence de la position 24 à la position 226 de la SEQ ID No. 5,
- un polypeptide se liant au facteur Willebrand et comprenant le motif peptidique $Z_1$-GPRGQPGVMGFP-$Z_2$- GPR-GQPGVMGFP et le motif peptidique $(GPP)_k$

dans lesquels indépendamment

$Z_1$ représente un linker comprenant de 6 à 12 acides aminés,

$Z_2$ représente un linker comprenant de 6 à 12 acides aminés,

k est un nombre entier compris entre 4 et 15.

De préférence, $Z_1$ et $Z_2$ représentent indépendamment un motif peptidique de formule $(GAA_1AA_2)_{n1}(GAA_3AA_4)_{n2}(GAA_5AA_6)_{n3}(GAA_7AA_8)_{n4}$

dans lequel indépendamment

$AA_1$, $AA_3$, $AA_5$ et $AA_7$ représentent indépendamment les acides aminés D ou A,

$AA_2$, $AA_4$, $AA_6$ et $AA_8$ représentent indépendamment les acides aminés A ou P,

$n_1$, $n_2$, $n_3$ et $n_4$ sont choisis indépendamment parmi 0, 1, 2, 3 ou 4 et la somme $n_1+n_2+n_3+n_4$ est égale à 2, 3 ou 4. Plus préférentiellement, $Z_1$ et $Z_2$ représentent le motif peptidique GDAGAPGAP de la SEQ ID No.7.

Les polypeptides selon la présente invention comprennent au moins deux motifs de liaison au facteur Willebrand de formule GPRGQPGVMGFP (SEQ ID No. 6) séparés par un linker. De tels linkers sont décrits ci-dessous.

De préférence, les polypeptides selon la présente invention comprennent un motif de type GPRGQPGVMGFP-Linker-GPRGQPGVMGFP et plus préférentiellement un motif de type « Linker -GPRGQPGVMGFP- Linker - GPRGQPGVMGFP - Linker ».

Dans un mode de réalisation préféré de l'invention, les polypeptides comprennent un motif de type $(GPP)_k$ - Linker -GPRGQPGVMGFP- Linker -GPRGQPGVMGFP ou un motif de type GPRGQPGVMGFP- Linker -GPR-GQPGVMGFP - Linker - $(GPP)_k$.

Dans un mode de réalisation de la présente invention, les polypeptides selon la présente invention comprennent le motif de la SEQ ID No. 8.

Toute séquence d'acides aminés peut être mise en oeuvre comme linker. De préférence, le linker comprend 6-12 acides aminés.

Dans un mode de réalisation préféré, le linker représente un motif peptidique de formule $(GAA_1AA_2)_{n1}(GAA_3AA_4)_{n2}(GAA_5AA_6)_{n3}(GAA_7AA_8)_{n4}$

dans lequel indépendamment

$AA_1$, $AA_3$, $AA_5$ et $AA_7$ représentent indépendamment les acides aminés D ou A,

$AA_2$, $AA_4$, $AA_6$ et $AA_8$ représentent indépendamment les acides aminés A ou P,

$n_1$, $n_2$, $n_3$ et $n_4$ sont choisis indépendamment parmi 0, 1, 2, 3 ou 4 et la somme $n_1+n_2+n_3+n_4$ est égale à 2, 3 ou 4. Plus préférentiellement, le linker représente le motif peptidique GDAGAPGAP de la SEQ ID No.7.

Le motif peptidique de formule $(GPP)_k$, dans laquelle k est un nombre entier compris entre 4 et 15, permet la trimérisation du polypeptide selon la présente invention. De préférence, dans la formule $(GPP)_k$, k est égal à 10. Ce motif detrimérisation est avantageusement situé à l'une des extrémités du polypeptide par rapport au motif de liaison au facteur Willebrand et séparé de ce dernier par un nombre variable d'acides aminés.

L'invention se rapporte également aux polypeptides ayant au moins 50%, 60%, 70%, 80%, 85%, 90%, 95% d'identité avec l'un des polypeptides suivants :

- le polypeptide de la SEQ ID No.2,
- le polypeptide de la position 24 à la position 184 de la SEQ ID No.2,
- le polypeptide de la SEQ ID No.4,
- le polypeptide de la position 24 à la position 205 de la SEQ ID No.4,
- le polypeptide de la SEQ ID No. 5,
- le polypeptide de la position 24 à la position 226 de la SEQ ID No. 5.

Par acides aminés identiques, on entend des acides aminés invariants ou inchangés entre deux séquences. Ces polypeptides peuvent présenter une délétion, une addition ou une substitution d'au moins un acide aminé par rapport au polypeptide de référence.

Par « pourcentage d'identité » entre deux séquences d'acides aminés au sens de la présente invention, on entend un pourcentage de résidus d'acides aminés identiques entre les deux séquences à comparer, obtenu après le meilleur alignement (alignement optimal), ce pourcentage étant purement statistique et les différences entre les deux séquences étant réparties au hasard et sur toute leur longueur. Ce pourcentage d'identité entre deux polypeptides peut être déterminé par un algorithme d'identité globale tel que décrit par Neddleman et Wunsch(1970).

De préférence, les polypeptides présentant un pourcentage d'identité avec les polypeptides de la présente invention conservent les propriétés du polypeptide de référence. De préférence, ces polypeptides conservent leur capacité de liaison au facteur Willebrand et leur solubilité dans l'eau notamment à pH physiologique.

Les polypeptides de la présente invention comprennent typiquement entre 150 et 300 acides aminés.

Pour leur mise en oeuvre dans des tests de liaison au facteur Willebrand les polypeptides de la présente invention peuvent être fixés sur tout type de support solide tel que des microplaques pour tests ELISA ou sur des billes dont les billes de latex ou les billes d'or.

L'invention a aussi pour objet des polynucléotides codant pour les polypeptides de la présente invention tels que le polynucléotide de la SEQ ID No. 1.

La présente invention a également pour objet un procédé pour déterminer l'activité de liaison du facteur Willebrand au collagène dans un échantillon biologique comprenant les étapes suivantes :

a) Mise à disposition d'un polypeptide choisi parmi :

- le polypeptide comprenant la séquence de la position 24 à la position 184 de la SEQ ID No.2,
- le polypeptide comprenant la séquence de la position 24 à la position 205 de la SEQ ID No.4,
- le polypeptide comprenant la séquence de la position 24 à la position 226 de la SEQ ID No. 5,
- un polypeptide se liant au facteur Willebrand et comprenant le motif peptidique $Z_1$-GPRGQPGVMGFP-$Z_2$-GPR-GQPGVMGFP et le motif peptidique $(GPP)_k$

dans lesquels indépendamment
$Z_1$ représente un linker comprenant de 6 à 12 acides aminés,
$Z_2$ représente un linker comprenant de 6 à 12 acides aminés,
k est un nombre entier compris entre 4 et 10 ;
b) Mise en contact de l'échantillon biologique avec ledit polypeptide ;
c) Mesure de la liaison du facteur Willebrand de l'échantillon biologique au polypeptide de l'étape a).

Dans la dernière étape du procédé, la mesure de la liaison du facteur Willebrand de l'échantillon biologique au polypeptide selon l'invention donne une mesure de la liaison du facteur Willebrand au collagène et notamment au collagène III humain.

Ce procédé peut être mis en oeuvre avec les polypeptides selon la présente invention tels que décrits ci-dessus.

L'invention se rapporte également à un procédé de diagnostic chez un patient souffrant ou suspecté de souffrir d'une forme de la maladie de Willebrand comprenant les étapes suivantes :

a) Mise à disposition d'un polypeptide choisi parmi :

- le polypeptide comprenant la séquence de la position 24 à la position 184 de la SEQ ID No.2,
- le polypeptide comprenant la séquence de la position 24 à la position 205 de la SEQ ID No.4,
- le polypeptide comprenant la séquence de la position 24 à la position 226 de la SEQ ID No. 5,
- un polypeptide se liant au facteur Willebrand et comprenant le motif peptidique $Z_1$-GPRGQPGVMGFP-$Z_2$-GPRGQPGVMGFP et le motif peptidique $(GPP)_k$

dans lesquels indépendamment
$Z_1$ représente un linker comprenant de 6 à 12 acides aminés,
$Z_2$ représente un linker comprenant de 6 à 12 acides aminés,
k est un nombre entier compris entre 4 et 10 ;
b) Mise en contact d'un échantillon biologique préalablement prélevé chez le patient avec ledit polypeptide ;
c) Mesure de la liaison du facteur Willebrand présent dans l'échantillon biologique au polypeptide de l'étape a).

L'invention se rapporte également à une méthode de diagnostic chez un patient souffrant ou suspecté de souffrir d'une forme de la maladie de Willebrand comprenant les étapes suivantes :

a) Prélèvement d'un échantillon biologique du patient,
b) Mise en contact de l'échantillon biologique prélevé chez le patient avec un polypeptide choisi parmi :

- le polypeptide comprenant la séquence de la position 24 à la position 184 de la SEQ ID No.2,
- le polypeptide comprenant la séquence de la position 24 à la position 205 de la SEQ ID No.4,
- le polypeptide comprenant la séquence de la position 24 à la position 226 de la SEQ ID No. 5,
- un polypeptide se liant au facteur Willebrand et comprenant le motif peptidique $Z_1$-GPRGQPGVMGFP-$Z_2$-GPRGQPGVMGFP et le motif peptidique $(GPP)_k$

dans lesquels indépendamment
$Z_1$ représente un linker comprenant de 6 à 12 acides aminés,
$Z_2$ représente un linker comprenant de 6 à 12 acides aminés,
k est un nombre entier compris entre 4 et 15 ;

c) Mesure de la liaison du facteur Willebrand présent dans l'échantillon biologique au polypeptide de l'étape a).

Dans les procédés de la présente invention, la dernière étape du procédé comprenant la mesure de la liaison du facteur Willebrand de l'échantillon biologique au polypeptide selon l'invention donne une mesure de la liaison du facteur Willebrand au collagène et notamment au collagène III humain.

Ces procédés peuvent être mis en oeuvre avec les polypeptides selon la présente invention tels que décrits ci-dessus.

Par échantillon, on entend notamment tout échantillon contenant ou susceptible de contenir le facteur Willebrand dont l'on cherche à déterminer l'activité de liaison au collagène et notamment au collagène de type III humain. Il peut notamment s'agir d'un échantillon biologique préalablement prélevé chez un patient. Dans des modes de réalisation préférés, l'échantillon biologique est du sang, du plasma, du plasma riche en plaquettes, un tissu biologique, un organe biologique ou un fluide corporel.

Les procédés selon la présente invention comportent une mise en contact de l'échantillon avec au moins un polypeptide selon la présente invention. Cette mise en contact est effectuée selon des méthodes classiques bien connues de l'homme du métier. Cette mise en contact s'effectue ainsi typiquement dans un milieu aqueux et plus préférentiellement dans un tampon garantissant la stabilité tridimensionnelle des polypeptides et dépourvu de protéases. Dans un mode de réalisation préféré, cette étape est réalisée dans un tampon phosphate.

Avantageusement, le polypeptide selon l'invention peut être fixé sur un support solide. Le polypeptide peut être fixé sur tout type de support naturel ou synthétique notamment tout polymère naturel ou synthétique. Ce support solide est par exemple choisi parmi le plastique, le polystyrène, le verre, le métal.

Dans un mode de réalisation, le polypeptide selon l'invention est fixé dans au moins un puits d'une microplaque de type plaque ELISA par exemple.

Dans un autre mode de réalisation, le polypeptide est fixé sur des billes de verre, de plastique, de polymère ou de métal, telles que des billes de latex ou d'or, par adsorption ou liaison covalente notamment.

La mesure de la liaison du facteur Willebrand de l'échantillon biologique au polypeptide selon l'invention est effectuée selon toute méthode appropriée. Ces méthodes sont bien connues de l'homme du métier. On citera en particulier, les méthodes ELISA ou les tests d'agglutination et d'agrégation et les tests en condition de flux.

Cette mesure peut être réalisée dans des conditions statiques en absence de toute agitation du milieu dans lequel s'effectue la liaison.

Dans d'autres modes de réalisation, la mesure de la liaison est réalisée en conditions de flux afin de reproduire les conditions de la circulation sanguine. Cette détermination permet de mesurer l'activité de liaison du facteur Willebrand à des collagènes humains ou animaux de type différent (types I, II, III, VI par exemple) dans les conditions physiologiques de la circulation sanguine. Des appareillages et des méthodes pour réaliser ces mesures sont bien connus de l'homme du métier comme la résonance plasmonique de surface.

La mesure de la liaison du facteur Willebrand aux polypeptides selon l'invention peut être suffisante pour établir un diagnostic de la maladie de Willebrand. En particulier, les procédés selon la présente invention permettent d'établir le diagnostic des types 1, 2, 2A, 2B, 2M et 3 de la maladie de Willebrand.

Dans certaines modes de réalisation, les procédés selon l'invention comprennent en outre une quantification du facteur Willebrand présent dans l'échantillon. Cette quantification peut s'effectuer selon toute technique habituelle. Dans un mode de réalisation, cette quantification du facteur Willebrand dans l'échantillon biologique est effectuée à l'aide d'anticorps dirigés contre le facteur Willebrand. Dans ce mode de réalisation, le procédé selon l'invention comprend une mesure de l'activité du facteur de Willebrand et plus particulièrement de son activité de liaison au collagène (dosage fonctionnel) et une mesure de la quantité de facteur Willebrand présent dans l'échantillon biologique (dosage quantitatif).

Classiquement, la mesure de l'activité de liaison du facteur Willebrand au collagène combiné avec la quantification du facteur Willebrand dans l'échantillon permet de déterminer le ratio :

$$R = \frac{facteur\ Willebrand\ lié\ au\ polypeptide\ (activité)}{facteur\ Willebrand\ présent\ dans\ l'échantillon\ biologique\ (antigène)}.$$

La détermination de ce ratio permet notamment d'établir un diagnostic ou d'affiner le diagnostic de la maladie de Willebrand et plus particulièrement de distinguer les types 1 et 3 (déficit quantitatif) et les types 2 (déficit qualitatif)de la maladie de Willebrand. Les procédés de la présente invention permettent également de distinguer et/ou d'établir un diagnostic des types 1, 2, 2A, 2B, 2M et 3 de la maladie de Willebrand.

L'invention se rapporte aussi à des kits pour déterminer l'activité de liaison du facteur Willebrand au collagène comprenant un polypeptide tel que décrit ci-dessus. Préférentiellement, le polypeptide est fixé sur un support solide tel qu'une bille de latex ou une bille d'or.

Le kit comprend en outre un réactif de détection pour déterminer la liaison du facteur Willebrand au collagène.

Ce réactif de détection peut être tout réactif habituellement mis en oeuvre. Il peut s'agir d'un marqueur tel un fluorophore fixé sur le polypeptide selon la présente invention. Dans un mode de réalisation, il s'agit d'un anticorps se fixant au polypeptide selon l'invention ou au facteur Willebrand. Cet anticorps peut être marqué afin de faciliter sa détection. De préférence, le kit comprend en outre un anticorps se liant au facteur Willebrand.

Figures

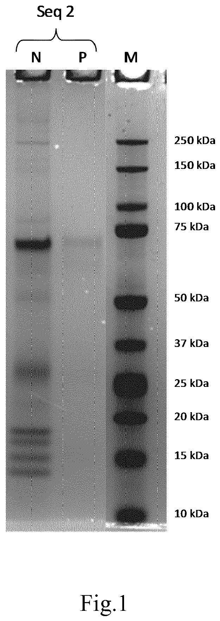

Figure 1 : Profil protéique représentatif des fractions purifiées du polypeptide selon l'invention (SEQ 2) digéré ou non à la pepsine. N = fraction normale non digérée, P = fraction digérée à la pepsine et M = marqueur de poids moléculaires. Dans la fraction N, nous observons une bande majoritaire à 70 kDa que l'on retrouve dans la fraction P. Cette bande correspond donc au polypeptide selon l'invention structuré en triple hélice.

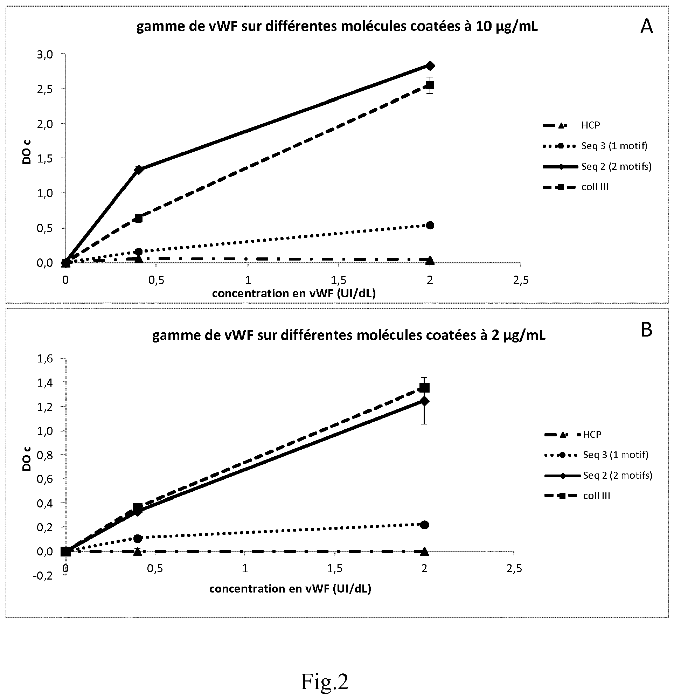

Figure 2 : Mesure de l'activité de liaison du facteur Willebrand purifié (Wilfactin) sur différents polypeptides selon l'invention possédant un motif (SEQ 3) ou deux motifs (SEQ 2) ainsi que sur le collagène de type III servant de référence (BD Biosciences) et sur des protéines endogènes ou HCP (Host CellProtein) servant de contrôle négatif. A = polypeptides coatés à 10 $\mu$g/mL et B = polypeptides coatés à 2 $\mu$g/mL. On observe qu'il faut au moins 2 motifs de liaison au facteur Willebrand pour obtenir une liaison du facteur Willebrand équivalente à celle du collagène de type III natif. Moyenne +/- ET avec n = 2

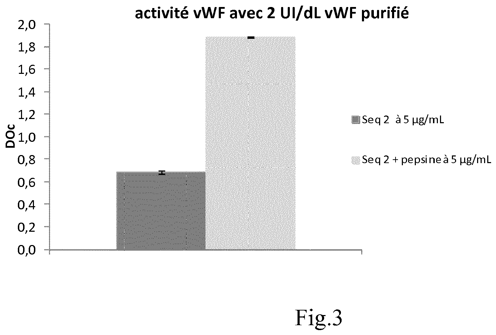

Figure 3 : Mesure de la liaison du facteur Willebrand (Wilfactin) à 2 UI/dL sur le polypeptide selon l'invention (Seq 2) digéré ou non par la pepsine et coaté à 2 $\mu$g/mL. On observe une augmentation d'un facteur 3 de l'activité de liaison du facteur Willebrand lorsque le polypeptide selon l'invention est digéré à la pepsine. Moyenne +/- ET avec n = 2

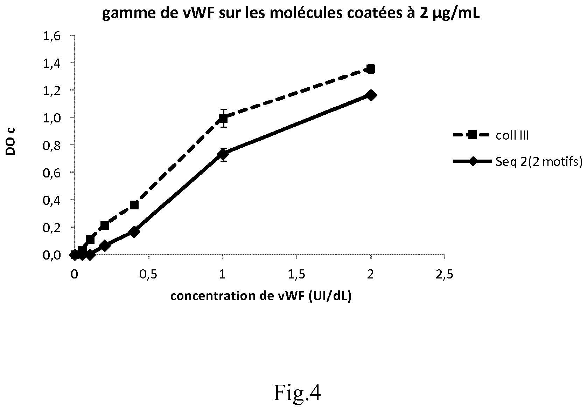

Figure 4 : Mesure de la liaison au polypeptide selon l'invention (SEQ 2) et au collagène de type III coatés à 2 $\mu$g/mL pour différentes concentrations en facteur Willebrand (0 ; 0,05 ; 0,1 ; 0,2 ; 0,4 ; 1 et 2 UI/dL). Le polypeptide selon l'invention possède la même activité de liaison du facteur Willebrand et la même sensibilité que le collagène III natif. Moyenne +/-ET avec n = 2

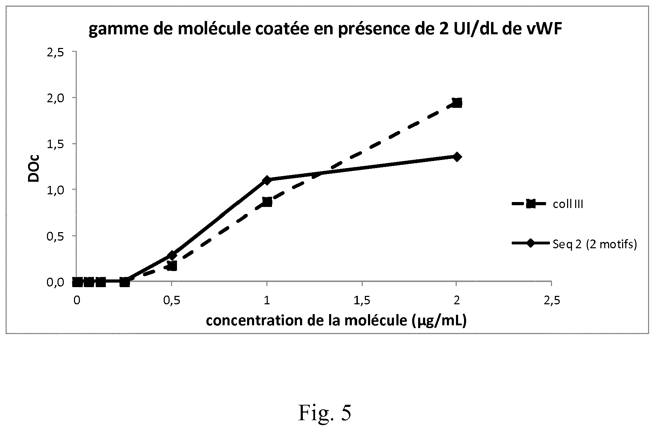

Figure 5 : Mesure de la liaison du facteur Willebrand utilisé à 2 UI/dL pour différentes concentrations de polypeptide selon l'invention (SEQ 2) et de collagène de type III (0 ; 0,032 ; 0,062 ; 0,125 ; 0,25 ; 0,5 ; 1 et 2 $\mu$g/mL). Moyenne +/- ET avec n = 2.

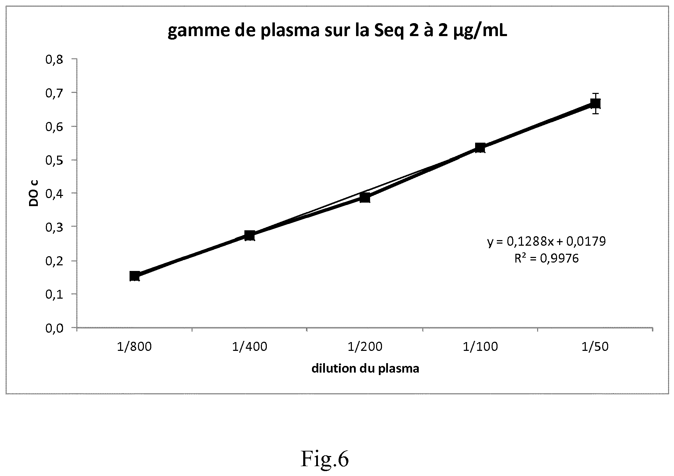

Figure 6 : Mesure de la liaison du facteur Willebrand plasmatique sur le polypeptide selon l'invention coaté à 2 $\mu$g/mL pour différentes dilutions d'un plasma témoin. Les résultats montrent une linéarité de la liaison du facteur Willebrand au polypeptide selon l'invention avec une courbe de régression linéaire présentant un $R^2$ à 0.9976. On observe également une bonne reproductibilité des résultats obtenus avec le polypeptide selon l'invention. Moyenne +/- ET avec n = 10.

Exemples

**Exemple 1. Construction du vecteur codant pour le polypeptide selon l'invention contenant une étiquette 6xHis**

La séquence nucléotidique codant pour les polypeptides selon l'invention est issue du vecteur pUC57-NVH020B contenant le peptide signal du collagène de type III (position 1 à 23 de la séquence nucléotidique du polypeptide selon l'invention). Cette séquence est insérée dans un vecteur d'expression dédié aux cellules de mammifère : pcDNA3.1 + (INVITROGEN). Ce vecteur contient, entre autre, les éléments suivants :

 o Un promoteur CMV,
 o Un site de clonage multiple pour y insérer la séquence nucléotidique d'intérêt,
 o Une cassette de résistance à la généticine pour l'expression en cellules de mammifère,
 o Une cassette de résistance à l'ampicilline pour l'expression en bactéries.

Pour permettre l'insertion du polypeptide selon l'invention, des sites de restriction ont été insérés de part et d'autre de la séquence nucléotidique. Il s'agit du site de restriction NheI en 5' et du site de restriction BamHI en 3'. Pour faciliter la purification du polypeptide selon l'invention, un tag 6 X histidine est ajouté en 5' ou en 3' de la séquence du polypeptidique. Afin de pouvoir retirer ce tag ultérieurement, une séquence de clivage à la TEV (TabaccoEtch Virus) a été insérée entre la séquence nucléotidique du tag 6 X histidine et la séquence codante pour le polypeptide selon l'invention.

Tous les plasmides finaux sont séquencés pour vérifier qu'aucune mutation ne s'est introduite dans l'ADN de la protéine d'intérêt et dans les éléments apportés.

**Exemple 2. Expression de la protéine de fusion selon l'invention dans les cellules CHO**

Une pré-culture des cellules CHO-S (INVITROGEN) de 3 semaines est réalisée avant la transfection. Les cellules sont entretenues dans un milieu dédié aux cellules CHO (Power-CHO, EXCEL 302, proCHO4, proCHO5, ...) complémenté avec 4 mM de L-glutamine (LONZA) et 1X de proHT (LONZA) en shakeflask 125 mL et en conditions agitées (80 rpm) dans un incubateur à 37°C avec 5% de $CO_2$. Deux jours avant la transfection, les cellules sont passées à $5.10^5$ cellules viables/mL par un changement complet du milieu utilisé et cultivées dans 12,5 mL de milieu dédié aux cellules CHO complet en shakeflask 125 mL.

Le jour de la transfection, $5.10^6$ cellules viables sont culotées par centrifugation (5 min à 1000 g), puis reprises dans 5 mL de milieu RPMI (Lonza) complémenté avec 4 mM de L-Glutamine (Lonza) et 1X de ProHT (Lonza). QuatremL de suspension sont alors répartis dans 4 shakeflasks 25 mL (1 mL par flask) contenant 9 mL de milieu RPMI complet ($1.10^6$ cellules viables par shakeflask). Les cellules CHO-S sont alors transfectées avec le vecteur contenant la séquence codante pour le polypeptide selon l'invention décrit précédemment. Un contrôle positif de transfection est réalisé en transfectant les cellules avec le vecteur pMAX-GFP et un contrôle négatif de la transfection est réalisé en transfectant les cellules avec un vecteur ne possédant pas la cassette de résistance à la généticine. La transfection est réalisée à l'aide de l'agent de transfection Fecturine (PolyPlus Transfection) ou tout autre agent de transfection adapté et selon le protocole commercial du produit optimisé. Pour la Fecturine, les conditions de transfection retenues sont 6 μg d'ADN pour 12 μL de Fecturine (ratio ADN/Agent de transfection = ½). L'homme de métier saura définir l'agent de transfection le plus adapté pour une transfection dite transitoire ou pour une transfection dite stable. Qu'il s'agisse d'un mode de transfection dit transitoire ou stable, les cellules sont incubées en présence des complexes de transfection en conditions statiques à 37°C et 5% $CO_2$. A 4h post-transfection, les cellules sont resuspendues dans du milieu dédié aux cellules CHO complet, et à 24h post-transfection, une quantification de l'efficacité de transfection est réalisée sur les témoins positifs et négatifs de transfection par cytométrie en flux.

Pour la production du polypeptide selon l'invention en mode dit transitoire, un premier prélèvement de surnageant est réalisé à J+3 puis la culture est stoppée à J+5. Le milieu contenant le polypeptide selon l'invention sécrété par les cellules est récupéré après centrifugation à 3 000 g pendant 10 minutes, permettant l'élimination des cellules, et congelé à -20°C.

Pour la production du polypeptide selon l'invention en mode dit stable, les cellules sont remises en suspension et un comptage cellulaire est réalisé à 48 heures post-transfection. La totalité des cellules est ensuite centrifugée à 1000 g pendant 5 min puis ensemencée à $3.10^5$ cellules viables/mL dans du milieu dédié aux cellules CHO complémenté + généticine (G418 Merck) à 700 μg/mL. Les cellules sont ensuite entretenues trois fois par semaine. La totalité des cellules est ensemencée dans 12,5 mL final de milieu complet + G418 en ShakeFlasks 125 mL. On observe une diminution de la concentration cellulaire et/ou de la viabilité cellulaire dans la première semaine de culture sous pression de sélection. La viabilité cellulaire augmente de nouveau après 2-3 semaines sous pression de sélection. Lorsque la culture atteint plus de 95 % de viabilité, une cryo-conservation à $5.10^6$ cellules viables/ampoule (au nombre de 10 ampoules) est réalisée. Les cellules sont congelées dans du milieu dédié aux cellules CHO + 20% de DMSO.

**Exemple 3. Production de la protéine de fusion selon l'invention dans les cellules CHO**

Les cellules modifiées génétiquement pour produire le polypeptide selon l'invention sont décongelées et entretenues dans un milieu adapté. Les cellules sont entretenues à $3.10^5$ cellules viables/mL pendant une à deux semaines. Les cellules sont placées dans 12,5 mL de milieu final et placées dans un shakeflask 125 mL dans un agitateur LabTherm® Kühner agité à 80 rpm, régulé à 5% de $CO_2$ et avec une humidité comprise entre 40 et 80 %. Ensuite, les cellules sont amplifiées afin d'avoir la quantité nécessaire pour réaliser une production. L'amplification consiste à entretenir les cellules dans des volumes plus élevés à chaque entretien pour garder toutes les cellules à une concentration viable.

Lorsque la quantité de cellules nécessaire à la production est obtenue, la production peut être lancée. Les cellules sont ensemencées à $3.10^5$ cellules viables/mL. La production peut être réalisée dans différents équipements : shakeflask placés dans un agitateur LabTherm® Kühner, Cultibag RM 20/50® (Sartorius), CellReady® (Merck Millipore), BioBundle® (Applikon) et d'autres équipements équivalents, de même échelle ou d'échelle supérieure. La culture des cellules est réalisée pendant 5 jours ou plus, au maximum 10 jours, selon les conditions de réalisation de la culture. Chaque jour, les paramètres de la production sont suivis ainsi que la concentration cellulaire et la viabilité cellulaire. Au cours de la production, des composants tels que des acides aminés, des vitamines, du glucose ou tout autre élément présentant un intérêt pour la production ou pour les cellules peuvent être ajoutés. Dans ce cas, il s'agit d'une culture en mode « fed - batch » ou « semi continu » qui permet de cultiver les cellules pendant au maximum 21 jours. Si aucun composé n'est ajouté pendant la culture, on parle de culture en mode « batch » ou « discontinu ».

**Exemple 4. Isolement par chromatographie d'affinité de la protéine de fusion**

Les cellules et débris cellulaires présents dans le milieu contenant le polypeptide selon l'invention sont éliminés par centrifugation ou filtration en profondeur (POD Millistak+® Merck Millipore ou tout un autre type de support équivalent) ou filtration tangentielle sur une membrane présentant un seuil de coupure de 0,2 μm. Le surnageant ainsi obtenu est purifié par chromatographie d'affinité sur une colonne chélatée avec un métal tel que le nickel, le cobalt, le zinc ou le cuivre. Afin de favoriser l'accroche du polypeptide selon l'invention, un tampon contenant de 0 à 50 mM imidazole, de 0 à 500 mMNaCl, de 5 à 20 mM ($Na_2HPO_4$, $2H_2O$) et de 5 à 20 mM ($NaH_2PO_4$, $H_2O$) est ajouté au surnageant et ajusté à un pH entre 7 et 8. L'élution du polypeptide selon l'invention est soit réalisée par gradient en utilisant un mélange de deux tampons (tampon 1: 500 mM imidazole, 500 mMNaCl, 10 mM ($Na_2HPO_4$, $2H_2O$) et 10 mM ($NaH_2PO_4$, $H_2O$) ; tampon 2 : 20 mM imidazole, 500 mMNaCl, 10 mM ($Na_2HPO_4$, $2H_2O$) et 10 mM ($NaH_2PO_4$, $H_2O$)), soit de façon isocratique, avec un tampon contenant de 50 à 500 mM imidazole, de 0 à 500 mMNaCl, de 5 à 10 mM ($Na_2HPO_4$, $2H_2O$)) et de 5 à 10 mM ($NaH_2PO_4$, $H_2O$). Eventuellement, afin d'améliorer la pureté du polypeptide selon l'invention, d'autres étapes de purification peuvent être ajoutées. Il peut s'agir de filtrations, de chromatographie d'échange d'ions, de chromatographie d'affinité, de chromatographie d'interaction hydrophobe, d'exclusion stérique ou de tout autre type de chromatographie.

Les fractions d'élution d'intérêt sont séparées dans un gel d'électrophorèse en conditions natives et colorées au bleu de Coomassie, avant d'être regroupées selon leur profil et dialysées dans de l'eau ou un tampon phosphate ou tout autre tampon adapté à la conservation du polypeptide selon l'invention. La concentration du polypeptide est déterminée par le kit Sircol® (TebuBio) selon les instructions du fabricant ou par le test au Bradford ou tout autre test adapté à la quantification du polypeptide de l'invention. Dans un certain mode de réalisation de l'invention, l'extrait protéique obtenu est digéré par la pepsine afin de déterminer si le polypeptide selon l'invention s'est structuré en triple hélice du fait de la présence, dans la séquence du polypeptide selon l'invention, d'une répétition de 10 fois du motif GPP, décrit comme permettant la trimérisation de protéines et peptides dérivés du collagène. Dans un mode privilégié de réalisation, la digestion par la pepsine est réalisée en incubant l'extrait protéique contenant le polypeptide selon l'invention pendant 5-6 heures dans un bain-marie à 37°C en présence de pepsine dans un ratio (concentration extrait protéique)/(concentration pepsine) de 20 pour 1. La pepsine est reconstituée dans un tampon 20 mM acétate de sodium ajusté à pH 4 avec de l'acide acétique. Le mélange extrait protéique/pepsine doit avoir un pH acide. Le pH est ajusté avec de l'acide acétique et validé sur du papier pH. Après digestion, la réaction est arrêtée avec 1/10 du volume de mélange réactionnel de 2 M Tris pH 11.4 afin d'obtenir une solution à pH basique. Le mélange réactionnel est ensuite passé ou non sur des cut off pour éliminer les fragments digérés, concentrer et/ou dialyser l'échantillon contre tout tampon adapté à la conservation du polypeptide selon l'invention ainsi digéré. La digestion des fractions est validée par migration de ces dernières sur un gel d'électrophorèse en conditions natives et coloré en bleu de Coomassie.

La figure 1 présente un profil d'électrophorèse montrant la présence du polypeptide selon l'invention dans le pool des différentes fractions obtenues après purification sur colonne d'affinité. La bande principale obtenue à un poids moléculaire autour de 70 kDa. On peut également observer la présence de bandes correspondant à des protéines de poids moléculaires de 14, 15, 17, 18, 30, 50, 60, 80, 150, 190, 250 et >250 kDa. A noter que le collagène est décrit pour ne pas migrer à la masse moléculaire attendue, ici environ 50 kDa pour le polypeptide de la SEQ 2 lorsqu'il est sous forme de trimère. La digestion par la pepsine de l'extrait protéique obtenu confirme que la bande à 70 kDa correspond bien au polypeptide selon l'invention structuré sous forme d'une triple-hélice résistante à la digestion par la pepsine. Le poids moléculaire de 70 kDa peut également s'expliquer par la présence de modifications post-traductionnelles au niveau des chaines alpha du polypeptide selon l'invention.

**Exemple 5. Méthode de détermination de l'activité de liaison du Willebrand au moyen d'un test ELISA**

L'un des modes de réalisation de la présente invention consiste en la mesure de l'activité de liaison du facteur Willebrand au polypeptide selon l'invention par la technique dite ELISA, réalisée dans des plaques 96 puits (Nunc Maxisorp). Pour cela, un volume de 100 μL d'une solution contenant le polypeptide à 2 ou 10 μg/mL dans du tampon phosphate ou tout autre tampon adapté est ajouté dans chaque puits et incubé 18-20h à 22°C. Après trois lavages avec 200 μL de PBS-0,05% Tween, 200 μL d'une solution de BSA 1% dans du tampon phosphate (Euromedex, filtrée sur 0,45 μm) sont ajoutés dans chaque puits et incubés 2h à température ambiante (22 °C). Après trois lavages avec 200 μL de PBS-0,05% Tween, 100 μL de différentes concentrations (exprimées en UI/dL) de facteur Willebrand purifié (Wilfactin 100 UI/mL, LFB) et/ou de plasma de patient dilué dans du tampon phosphateou tout autre tampon adapté sont incubés 1h30 à température ambiante (22°C). Après trois nouveaux lavages, 100 μL d'une solution contenant un anticorps primaire anti-vWF couplé à la peroxydase de raifort (Rabbit anti-human VWF/HRP, DAKO) dilué au 1/8 000e dans du tampon phosphate ou tout autre tampon adapté sont incubés 1h à 22°C puis 4 lavages sont réalisés comme décrit ci-dessus. On ajoute ensuite 125 μL de solution de tétraméthylbenzidine comme substrat pour la peroxydase (TMB, Sigma) puis on incube 10 à 45 minutes maximum à l'obscurité. La réaction est interrompue par l'ajout de 125 μL

d'HCl 2N (Sigma). L'absorbance est rapidement mesurée à 450 nm dans un spectrophomètre (Wallacvictor 3). Un blanc est réalisé en remplaçant le vWF purifié ou le plasma par du tampon phosphate ou tout autre tampon adapté.

La figure 2 présente un résultat obtenu par ELISA de la liaison du facteur Willebrand purifié (Willfactin, LFB) utilisé à 0 ; 0,4 et 2 UI/dL finale aux polypeptides selon l'invention correspondant à la SEQ 2, à la SEQ 3, au collagène de type III (BD Biosciences, contrôle positif) et aux HCP (Host CellProteins, obtenues de la même façon que les polypeptides selon l'invention à partir de cellules CHO non transfectées, contrôle négatif), contenant respectivement deux (SEQ 2) et un (SEQ 3) motifs de reconnaissance au facteur Willebrand. Ces différents polypeptides sont utilisés à 10 ou 2 μg/mL (Fig. 2A et 2B). Les résultats montrent que seul le polypeptide selon l'invention contenant au moins deux motifs (SEQ 2) de reconnaissance au facteur Willebrand est capable de lier ce facteur de façon équivalente au collagène de type III de référence.

La figure 3 présente un résultat obtenu par ELISA de la liaison du facteur Willebrand purifié (Willfactin, LFB) utilisé à 2 UI/dL au polypeptide selon l'invention (SEQ 2) après digestion ou non par la pepsine et obtention d'une fraction pure contenant la forme en triple-hélice du polypeptide selon l'invention. La liaison du Willebrand est mesurée pour une concentration de 5 μg/mL de chaque polypeptide. Les résultats montrent une forte augmentation de la capacité de liaison du polypeptide selon l'invention lorsqu'il est uniquement sous forme d'une triple-hélice. Ces résultats indiquent que le traitement à la pepsine permet de digérer les contaminants protéiques éventuels, issus des cellules CHO-S et capables de se lier à la colonne d'affinité à base de nickel ou de cobalt, et/ou de digérer les polypeptides selon l'invention non structurés en triple-hélice. Ce résultat suggère également que c'est la forme en triple hélice qui porte l'activité du polypeptide selon l'invention.

La figure 4 présente la mesure de la liaison pour différentes concentrations en facteur Willebrand (0 ; 0,05 ; 0,1 ; 0,2 ; 0,4 ; 1 et 2 UI/dL) du polypeptide selon l'invention (SEQ 2) et du collagène de type III coatés à 2 μg/mL. On observe que le polypeptide selon l'invention a une sensibilité équivalente à celle du collagène de type III pour les faibles concentrations en Willebrand. Ce point est particulièrement important car l'objectif du test utilisant le polypeptide selon l'invention est de déterminer une activité pour des valeurs plasmatiques en Willebrand très faibles, telles qu'observées pour les patients du type 2.

La figure 5 présente un résultat obtenu par ELISA de la liaison du facteur Willebrand purifié (Willfactin, LFB), utilisé à 2 UI/dL au polypeptide selon l'invention (SEQ 2) et au collagène de type III utilisés à 2; 1; 0,5; 0,25; 0,125; 0,0625 et 0,03125μg/mL. Les résultats confirment que le polypeptide selon l'invention est capable de lier le facteur Willebrand de façon équivalente au collagène de type III utilisé comme référence avec la même sensibilité.

L'application du polypeptide selon l'invention porte sur la mesure de l'activité de liaison au facteur Willebrand présent dans le plasma de patients sains ou porteurs de la maladie de Willebrand. La figure 6 présente la mesure de la liaison du facteur Willebrand plasmatique au polypeptide selon l'invention pour différentes dilutions d'un plasma de référence, soit au 1/50ᵉ, au 1/100ᵉ, au 1/200ᵉ, au 1/400ᵉ et au 1/800ᵉ. Ces dilutions sont celles classiquement utilisées pour la détermination de la courbe standard obtenue à partir d'un plasma de référence et indispensable pour le calcul de l'activité de liaison du collagène au Willebrand pour un patient donné. Les résultats montrent la linéarité de la liaison du facteur Willebrand plasmatique au polypeptide selon l'invention avec une droite de régression linéaire possédantun coefficient de corrélation $R^2$ égal à 0.9976.

La mesure de la liaison du facteur Willebrand au collagène doit permettre de faciliter la classification des patients dans les différents groupes de la maladie de Willebrand. Le tableau I présente les valeurs obtenues par ELISA de plasmas de patients appartenant au type I, au type II ou avec suspicion de la présence d'anticorps anti-vWF (mimant un type III) *vs* des patients sains et le rapport mesure d'activité (CollagenBinding)/mesure antigénique (Ag). On constate que ce rapport est bien supérieur à 0,7 pour les patients du type I, inférieur à 0,7 pour les patients du type II, et que les patients de type III ou possédant des anticorps anti-vWF ont des % CB et %Ag quasi nulsconfirmant l'intérêt du polypeptide selon l'invention pour la classification de ces patients. A noter que le polypeptide selon l'invention distingue mieux les patients de type I et II que le collagène de type III (100% de corrélation avec les données cliniques *versus*78%).

Tableau I : Classification des patients sains et pathologiques en fonction de leur ratio facteur Willebrand CB/Ag (mesure d'activité (CollagenBinding)/mesure antigénique (Ag)), la mesure étant obtenue soit sur le polypeptide ayant la ou de séquence SEQ 2, soit sur le collagène de type III servant de référence (BD Biosciences) coatés à 2 μg/mL. Le polypeptide de séquence SEQ 2 permet de distinguer les patients de type I des patients de type II et ce, avec un taux de corrélation aux données cliniques meilleur qu'avec le collagène de type III (100% *versus* 78%).

| patients | Seq 2 | | | | | | Collagène III | | | | | |
|---|---|---|---|---|---|---|---|---|---|---|---|---|
| | % CB moy | % AG | ratio CB/Ag | interprétation | classification | corrélation | % CB moy | % AG | ratio Co/Ag | interprétation | classification | corrélation |
| patient 1 | 150 | 117 | 1,28 | **normal** | normal | oui | 164 | 117 | 1,40 | **normal** | normal | oui |
| patient 2 | 150 | 86 | 1,74 | **normal** | normal | oui | 105 | 86 | 1,22 | **normal** | normal | oui |
| patient 3 | 59 | 45 | 1,31 | **type 1** | type 1 | oui | 37 | 45 | 0,82 | **type 1** | type 1 | oui |
| patient 4 | 22 | 17 | 1,29 | **type 1** | type 1 | oui | 24 | 17 | 1,41 | **type 1** | type 1 | oui |
| patient 5 | 33 | 22 | 1,50 | **type 1** | type 1 | oui | 17 | 22 | 0,77 | **type 1** | type 1 | oui |
| patient 6 | 11 | 23 | 0,48 | **type 2** | type 2 | oui | 17 | 23 | 0,74 | **type 1** | type 2 | **non** |
| patient 7 | 20 | 40 | 0,50 | **type 2** | type 2 | oui | 19 | 40 | 0,48 | **type 2** | type 2 | oui |
| patient 8 | 16 | 27 | 0,59 | **type 2** | type 2 | oui | 24 | 27 | 0,89 | **type 1** | type 2 | **non** |
| patient 9 | 1 | 5 | 0,20 | **Ac vWF** | Ac vWF | oui | 0 | 5 | 0,00 | **Ac vWF** | Ac vWF | oui |
| corrélation | 100,0% | | | | | | 78,0% | | | | | |

L'intérêt majeur de la mesure de la liaison du facteur Willebrand au collagène VWF:CB (Von WillebrandFactor:CollagenBinding), par opposition à la mesure de sa liaison à GpIb après activation par la ristocétine, réside dans le fait que ce dosage est très sensible à la perte des multimères de haut poids moléculaire, éléments déterminant de l'activité du facteur Willebrand. Il permet alors de discriminer des patients du type 2 et notamment les sous-types 2A et 2M.

Une étude récente a démontré l'intérêt de l'utilisation de la N-acétylcystéine (NAC) dans le traitement du purpura thrombotique thrombocytopénique (PTT) consécutive à l'adhésion des plaquettes à des multimères de facteur Willebrand de très grandes tailles (Chen J et coll., J Clin Invest. 2011 121(2) :593-603). Ces multimères sont la conséquence d'un déficit dans l'enzyme de maturation du facteur Willebrand, l'ADAMTS13 *(adisintegrinand metalloproteinasewithathrombospondin type 1 motif, member 13)*. Le traitement d'un pool de plasmas par le NAC conduit à la perte des multimères de hauts poids moléculaires et est associé à une diminution de la liaison du Willebrand au collagène.

**Exemple 6. Comparaison du test ELISA selon l'invention et de deux trousses ELISA commerciales (à base de collagène) dans le cadre d'une étude clinique multicentrique**

Comme décrit précédemment, l'une des utilisations de la présente invention consiste en la mesure de l'activité de liaison du facteur Willebrand au polypeptide selon l'invention par la technique dite ELISA. Afin de valider ce test en regard de trousses ELISA à base de collagène déjà commercialisées, une étude clinique multicentrique (6 centres ; autorisation 2012/58 - ID RCB N°2012-A01537-36) incluant 121 patients a été réalisée. Les plasmas collectés proviennent de patients se répartissant comme suit :

- 31 sujets témoins,
- 28 patients avec déficit type 1,
- 9 patients avec déficit type 3,
- 18 patients avec déficit type 2A,
- 17 patients avec déficit type 2B,
- 18 patients avec déficit type 2M.

Les trousses ELISA commerciales retenues pour cette étude contiennent soit du collagène de type I (TECHNOZYM® vWF:CBA ELISA Collagen type I; ref: 5450311 ; TechnocloneGmbH), soit du collagène de type III (ASSERACHROM® VWF :CB ; ref: 00239 ; Stago). La mesure de l'activité de liaison au facteur Willebrand pour ces deux trousses a été réalisée selon les instructions fournies par le fournisseur.

Concernant le test ELISA à base du polypeptide selon l'invention, il a été réalisé à une concentration finale de 2 $\mu$g/mL du polypeptide ayant la ou de séquence SEQ ()2 et la mesure effectuée sur des plasmas dilués au 1/400 en suivant le protocole décrit précédemment. La gamme standard utilisée pour la détermination de l'activité de liaison au facteur Willebrand a été obtenue à partir du plasma standard de la trousse ASSERACHROM® VWF :CB.

Les résultats présentés dans le tableau II montrent les moyennes et écarts types obtenues pour chacun des tests. Une bonne corrélation a été observée entre les valeurs d'activité des plasmas normaux et pathologiques obtenues avec le test ELISA utilisant le polypeptide selon l'invention et celles obtenues avec les trousses commerciales.

Tableau II : Mesure de l'activité de liaison du facteur Willebrand au collagène pour chacun des ELISA en fonction du type de déficit.

| | CBA Stago Coll type III | CBA Cryopep Coll type I | ELISA Polypeptide SEQ 2 2 µg/mL |
|---|---|---|---|
| Patients (nombre) | Moyenne (ET) | Moyenne (ET) | Moyenne (ET) |
| Témoins (31) | 103 (22.1) | 161 (40.7) | 98.1 (28.1) |
| Type 1 (28) | 23.9 (8.9) | 22.2 (11.7) | 19.7 (7.5) |
| Type 3 (9) | 1 (0.3) | 0.2 (0.4) | 1.6 (2.1) |
| Type 2A (18) | 15.0 (8.8) | 10.0 (10.8) | 16.9 (11.3) |
| Type 2B (17) | 29.5 (17.1) | 31.4 (26.6) | 31.8 (19.5) |
| Type 2M (18) | 21.8 (13.1) | 15.9 (19.8) | 22.5 (17.7) |

Ces résultats renforcent les données concernant la capacité du polypeptide selon l'invention à :

- lier le facteur Willebrand de façon équivalente à des collagènes natifs,
- discriminer des patients porteurs d'un déficit en facteur Willebrand par rapport à des témoins,
- être utilisé dans un test ELISA en substitution des collagènes natifs.

REFERENCES

REFERENCES BREVET

WO2010034718
WO2007052067
EP 1 870 460
EP 2383338

REFERENCES NON-BREVET

Flood VH, J Thromb Haemost.2012 Apr 16
Springer TA, J ThrombHaemost. 2011 Jul;9Suppl 1:130-43
Schneppenheim R, Thromb Res.2011;128 Suppl 1:S3-7
Chen C etRaghunath M. Fibrogenesis Tissue Repair. 2009 Dec 15;2:7
Herr AB et Farndale RW, J Biol Chem. 2009 Jul 24;284(30):19781-5
Lisman T et coll., Blood. 2006 Dec 1; 108(12) : 3753-6
Xu H et coll., Matrix Biol. 2011 Jan ; 30(1) :16-26
Giudici C et coll., J Biol Chem.2008 Jul 11;283(28):19551-60
Brondijk TH et coll. ProcNatlAcadSci U S A.2012 Apr 3;109(14):5253-8
Verkleijet coll., Blood. 1998 May 15; 91(10) : 3808-16
BonnefoyAet coll. J ThrombHaemost. 2006 Oct;4(10):2151-61
Favaloro EJ, Thromb Haemost.2010 Nov;104(5):1009-21
Baronciani L et coll. J Thromb Haemost.2006 Sep;4(9):2088-90
Heemskerk JWM et coll. J Thromb Haemost.2011 Apr;9(4):856-8
Werkmeister J etRamshaw J. Biomed Mater. 2012 Feb;7(1):012002
Chen J et coll., J Clin Invest.2011 121(2) :593-603
Neddlemanet Wunsch, J Mol Biol 1970 48:443

**Revendications**

1. Procédé de diagnostic de la maladie de Willebrand chez un patient **caractérisé en ce qu'**il comprend les étapes suivantes :

   a) Mise à disposition d'un polypeptide choisi parmi :

   - le polypeptide comprenant la séquence de la position 24 à la position 184 de la SEQ ID No.2,
   - le polypeptide comprenant la séquence de la position 24 à la position 205 de la SEQ ID No.4,
   - le polypeptide comprenant la séquence de la position 24 à la position 226 de la SEQ ID No. 5,
   - un polypeptide se liant au facteur Willebrand et comprenant le motif peptidique $Z_1$-GPRGQPGVMGFP-$Z_2$-GPRGQPGVMGFP et le motif peptidique $(GPP)_k$

   dans lesquels indépendamment
   $Z_1$ représente un linker comprenant de 6 à 12 acides aminés,
   $Z_2$ représente un linker comprenant de 6 à 12 acides aminés,
   k est un nombre entier compris entre 4 et 15 ;
   b) Mise en contact d'un échantillon biologique préalablement prélevé chez le patient avec ledit polypeptide ;
   c) Mesure de la liaison du facteur Willebrand présent dans l'échantillon biologique au polypeptide de l'étape a) pour mesurer l'activité de liaison du facteur Willebrand au collagène.

2. Procédé de diagnostic de la maladie de Willebrand chez un patient selon la revendication 1 **caractérisé en ce que** $Z_1$ et $Z_2$ représentent indépendamment un motif peptidique de formule $(GAA_1AA_2)_{n1}(GAA_3AA_4)_{n2}(GAA_5AA_6)_{n3}(GAA_7AA_8)_{n4}$ dans lequel indépendamment
   $AA_1$, $AA_3$, $AA_5$ et $AA_7$ représentent indépendamment les acides aminés D ou A,
   $AA_2$, $AA_4$, $AA_6$ et $AA_8$ représentent indépendamment les acides aminés A ou P,
   $n_1$, $n_2$, $n_3$ et $n_4$ sont choisis indépendamment parmi 0, 1, 2, 3 ou 4 et la somme $n_1+n_2+n_3+n_4$ est égale à 2, 3 ou 4.

3. Procédé de diagnostic de la maladie de Willebrand chez un patient selon l'une des revendications 1-2 **caractérisé en ce que** $Z_1$ et $Z_2$ représentent le motif peptidique GDAGAPGAP de la SEQ ID No.7.

4. Procédé de diagnostic de la maladie de Willebrand selon l'une des revendications 1-3 **caractérisé en ce qu'**il comprend en outre la détermination de la quantité de facteur Willebrand présent dans l'échantillon.

5. Procédé de diagnostic selon l'une des revendications 1-4 **caractérisé en ce que** le polypeptide de l'étape a) est fixé sur un support solide.

6. Procédé de diagnostic selon l'une des revendications 1-5 **caractérisé en ce que** les étapes b) et c) sont réalisés en conditions de flux.

7. Polypeptide **caractérisé en ce qu'**il est choisi parmi :

   - le polypeptide comprenant la séquence de la SEQ ID No.2,
   - le polypeptide comprenant la séquence de la position 24 à la position 184 de la SEQ ID No.2,

- le polypeptide comprenant la séquence de la SEQ ID No.4,
- le polypeptide comprenant la séquence de la position 24 à la position 205 de la SEQ ID No.4,
- le polypeptide comprenant la séquence de la SEQ ID No. 5,
- le polypeptide comprenant la séquence de la position 24 à la position 226 de la SEQ ID No. 5,
- un polypeptide se liant au facteur Willebrand et comprenant le motif peptidique $Z_1$-GPRGQPGVMGFP-$Z_2$-GPR-GQPGVMGFP et le motif peptidique $(GPP)_k$

dans lesquels indépendamment
$Z_1$ représente un linker comprenant de 6 à 12 acides aminés,
$Z_2$ représente un linker comprenant de 6 à 12 acides aminés,
k est un nombre entier compris entre 4 et 15.

8. Kit pour déterminer l'activité de liaison du facteur Willebrand au collagène **caractérisé en ce qu'**il comprend :

a) un polypeptide selon la revendication 7 ;
b) un réactif de détection pour déterminer la liaison du facteur Willebrand au collagène.

9. Kit selon la revendication 8 **caractérisé en ce qu'**il comprend un anticorps se liant au facteur Willebrand.

**Patentansprüche**

1. Diagnoseverfahren des Willebrand-Syndroms bei einem Patienten, **dadurch gekennzeichnet, dass** es die folgenden Schritte umfasst:

a) Bereitstellen eines Polypeptids, ausgewählt aus:

- dem Polypeptid, umfassend die Sequenz der Position 24 bis zur Position 184 der SEQ ID Nr. 2,
- dem Polypeptid, umfassend die Sequenz der Position 24 bis zur Position 205 der SEQ ID Nr. 4,
- dem Polypeptid, umfassend die Sequenz der Position 24 bis zur Position 226 der SEQ ID Nr. 5,
- einem Polypeptid, das sich an den Willebrand-Faktor bindet und das Peptidmotiv $Z_1$-GPRGQPGVMGFP-$Z_2$-GPRGQPGVMGFP und das Peptidmotiv $(GPP)_k$ umfasst,

in welchen unabhängig
$Z_1$ einen Linker mit 6 bis 12 Aminosäuren darstellt,
$Z_2$ einen Linker mit 6 bis 12 Aminosäuren darstellt,
k eine Ganzzahl zwischen 4 und 15 inklusive ist,
b) Inkontaktversetzen einer biologischen Probe, die zuvor einem Patienten entnommen wurde, mit dem Polypeptid,
c) Messen der Bindung des in der biologischen Probe vorhandenen Willebrand-Faktors an das Polypeptid von Schritt a), um die Aktivität der Bindung des Willebrand-Faktors an Kollagen zu messen.

2. Diagnoseverfahren des Willebrand-Syndroms bei einem Patienten nach Anspruch 1, **dadurch gekennzeichnet, dass** $Z_1$ und $Z_2$ unabhängig ein Peptidmotiv der Formel $(GAA_1AA_2)_{n1}(GAA_3AA_4)_{n2}(GAA_5AA_6)_{n3}(GAA_7AA_8)_{n4}$ darstellen, in welchem unabhängig
$AA_1$, $AA_3$, $AA_5$ und $AA_7$ unabhängig die Aminosäuren D oder A darstellen,
$AA_2$, $AA_4$, $AA_6$ und $AA_8$ unabhängig die Aminosäuren A oder P darstellen,
$n_1$, $n_2$, $n_3$ und $n_4$ unabhängig aus 0, 1, 2, 3 oder 4 ausgewählt sind und die Summe $n_1+n_2+n_3+n_4$ gleich 2, 3 oder 4 ist.

3. Diagnoseverfahren des Willebrand-Syndroms bei einem Patienten nach einem der Ansprüche 1-2, **dadurch gekennzeichnet, dass** $Z_1$ und $Z_2$ das Peptidmotiv GDAGAPGAP der SEQ ID Nr. 7 darstellen.

4. Diagnoseverfahren des Willebrand-Syndroms nach einem der Ansprüche 1-3, **dadurch gekennzeichnet, dass** es ferner die Bestimmung der Menge Willebrand-Faktor in der Probe umfasst.

5. Diagnoseverfahren nach einem der Ansprüche 1-4, **dadurch gekennzeichnet, dass** das Polypeptid von Schritt a) auf einem festen Träger fixiert ist.

6. Diagnoseverfahren nach einem der Ansprüche 1-5, **dadurch gekennzeichnet, dass** die Schritte b) und c) unter Flussbedingungen durchgeführt werden.

7. Polypeptid, **dadurch gekennzeichnet, dass** es ausgewählt ist aus:

   - dem Polypeptid, umfassend die Sequenz der SEQ ID Nr. 2,
   - dem Polypeptid, umfassend die Sequenz der Position 24 bis zur Position 184 der SEQ ID Nr. 2,
   - dem Polypeptid, umfassend die Sequenz der SEQ ID Nr. 4,
   - dem Polypeptid, umfassend die Sequenz der Position 24 bis zur Position 205 der SEQ ID Nr. 4,
   - dem Polypeptid, umfassend die Sequenz der SEQ ID Nr. 5,
   - dem Polypeptid, umfassend die Sequenz der Position 24 bis zur Position 226 der SEQ ID Nr. 5,
   - einem Polypeptid, das sich an den Willebrand-Faktor bindet und das Peptidmotiv $Z_1$-GPRGQPGVMGFP-$Z_2$-GPRGQPGVMGFP und das Peptidmotiv $(GPP)_k$ umfasst,

   in welchen unabhängig
   $Z_1$ einen Linker mit 6 bis 12 Aminosäuren darstellt,
   $Z_2$ einen Linker mit 6 bis 12 Aminosäuren darstellt,
   k eine Ganzzahl zwischen 4 und 15 inklusive ist.

8. Kit zur Bestimmung der Aktivität der Bindung des Willebrand-Faktors an Kollagen, **dadurch gekennzeichnet, dass** es umfasst:

   a) ein Polypeptid nach Anspruch 7,
   b) ein Detektionsreagenz zur Bestimmung der Bindung des Willebrand-Faktors an Kollagen.

9. Kit nach Anspruch 8, **dadurch gekennzeichnet, dass** es einen Antikörper umfasst, der sich an den Willebrand-Faktor bindet.

**Claims**

1. A method for diagnosing von Willebrand disease in a patient, **characterized in that** it comprises the following steps:

   a) Providing a polypeptide selected from:

   - the polypeptide comprising the sequence from position 24 to position 184 of SEQ ID NO: 2,
   - the polypeptide comprising the sequence from position 24 to position 205 of SEQ ID NO: 4,
   - the polypeptide comprising the sequence from position 24 to position 226 of SEQ ID NO: 5,
   - a von Willebrand factor-binding polypeptide comprising the peptide motif $Z_1$-GPRGQPGVMGFP-$Z_2$-GPRGQPGVMGFP and the peptide motif $(GPP)_k$

   wherein independently
   $Z_1$ represents a linker comprising 6 to 12 amino acids,
   $Z_2$ represents a linker comprising 6 to 12 amino acids,
   k is an integer between 4 and 15;
   b) Contacting a biological sample previously taken from the patient with said polypeptide;
   c) Measuring the binding of von Willebrand factor present in the biological sample to the polypeptide of step a) in order to measure von Willebrand factor collagen-binding activity.

2. The method for diagnosing von Willebrand disease in a patient according to claim 1, **characterized in that** $Z_1$ and $Z_2$ independently represent a peptide motif of formula $(GAA_1AA_2)_{n1}(GAA_3AA_4)_{n2}(GAA_5AA_6)_{n3}(GAA_7AA_8)_{n4}$
   wherein independently
   $AA_1$, $AA_3$, $AA_5$ and $AA_7$ independently represent amino acids D or A,
   $AA_2$, $AA_4$, $AA_6$ and $AA_8$ independently represent amino acids A or P,
   $n_1$, $n_2$, $n_3$ and $n_4$ are independently selected from 0, 1, 2, 3 or 4 and the sum $n_1+n_2+n_3+n_4$ is equal to 2, 3 or 4.

3. The method for diagnosing von Willebrand disease in a patient according to one of claims 1-2, **characterized in that** $Z_1$ and $Z_2$ represent the peptide motif GDAGAPGAP of SEQ ID NO: 7.

4. The method for diagnosing von Willebrand disease according to one of claims 1-3, **characterized in that** it further comprises determining the quantity of von Willebrand factor present in the sample.

5. The method for diagnosing according to one of claims 1-4, **characterized in that** the polypeptide of step a) is attached to a solid support.

6. The method for diagnosing according to one of claims 1-5, **characterized in that** steps b) and c) are carried out in flow conditions.

7. A polypeptide **characterized in that** it is selected from:

- the polypeptide comprising the sequence of SEQ ID NO: 2,
- the polypeptide comprising the sequence from position 24 to position 184 of SEQ ID NO: 2,
- the polypeptide comprising the sequence of SEQ ID NO: 4,
- the polypeptide comprising the sequence from position 24 to position 205 of SEQ ID NO: 4,
- the polypeptide comprising the sequence of SEQ ID NO: 5,
- the polypeptide comprising the sequence from position 24 to position 226 of SEQ ID NO: 5,
- a von Willebrand factor-binding polypeptide comprising the peptide motif $Z_1$-GPRGQPGVMGFP-$Z_2$-GPRGQPGVMGFP and the peptide motif $(GPP)_k$

wherein independently
$Z_1$ represents a linker comprising 6 to 12 amino acids,
$Z_2$ represents a linker comprising 6 to 12 amino acids,
k is an integer between 4 and 15.

8. A kit for determining von Willebrand factor collagen-binding activity, **characterized in that** it comprises:

a) a polypeptide according to claim 7;
b) a detection reagent for determining von Willebrand factor collagen-binding.

9. The kit according to claim 8, **characterized in that** it comprises a von Willebrand factor-binding antibody.

Fig.1

Fig.2

Fig.3

Fig.4

Fig. 5

Fig.6

## RÉFÉRENCES CITÉES DANS LA DESCRIPTION

**Documents brevets cités dans la description**

- EP 2383338 A
- WO 2010034718 A
- WO 2007052067 A
- EP 1870460 A
- FR 1259996

**Littérature non-brevet citée dans la description**

- **SPRINGER TA.** *J ThrombHaemost.,* Juillet 2011, vol. 9 (1), 130-43
- **SCHNEPPENHEIM R.** *Thromb Res.,* 2011, vol. 128 (1), 3-7
- **CHEN C ; RAGHUNATH M.** *Fibrogenesis Tissue Repair,* 15 Décembre 2009, vol. 2, 7
- **HERR AB ; FARNDALE RW.** *J BiolChem.,* 24 Juillet 2009, vol. 284 (30), 19781-5
- **LISMAN T.** *Blood,* 01 Décembre 2006, vol. 108 (12), 3753-6
- **GIUDICI C.** *J BiolChem.,* 11 Juillet 2008, vol. 283 (28), 19551-60
- **BRONDIJK TH.** *Proc NatlAcadSci U S A,* 03 Avril 2012, vol. 109 (14), 5253-8
- **VERKLEIJ.** *Blood,* 15 Mai 1998, vol. 91 (10), 3808-16
- **BONNEFOY A.** *J ThrombHaemost,* Octobre 2006, vol. 4 (10), 2151-61
- **FAVALORO EJ.** *Thromb Haemost.,* Novembre 2010, vol. 104 (5), 1009-21
- **FAVALORO EJ.** *Thromb Haemost,* Novembre 2010, vol. 104 (5), 1009-21
- **BARONCIANI L.** *J Thromb Haemost,* Septembre 2006, vol. 4 (9), 2088-90
- **HEEMSKERK JWM.** *J Thromb Haemost,* Avril 2011, vol. 9 (4), 856-8
- **WERKMEISTER J ; RAMSHAW J.** *Biomed Mater,* Février 2012, vol. 7 (1), 012002
- **CHEN J.** *J Clin Invest.,* 2011, vol. 121 (2), 593-603
- **FLOOD VH.** *J Thromb Haemost.,* 16 Avril 2012
- **SPRINGER TA.** *J ThrombHaemost,* Juillet 2011, vol. 9 (1), 130-43
- **CHEN C ; RAGHUNATH M.** *Fibrogenesis Tissue Repair,* 15 Décembre 2009, vol. 2, 7
- **HERR AB ; FARNDALE RW.** *J Biol Chem.,* 24 Juillet 2009, vol. 284 (30), 19781-5
- **XU H.** *Matrix Biol.,* Janvier 2011, vol. 30 (1), 16-26
- **GIUDICI C.** *J Biol Chem.,* 11 Juillet 2008, vol. 283 (28), 19551-60
- **BRONDIJK TH.** *ProcNatlAcadSci U S A,* 03 Avril 2012, vol. 109 (14), 5253-8
- **VERKLEIJET.** *Blood,* 15 Mai 1998, vol. 91 (10), 3808-16
- **BONNEFOYA.** *J ThrombHaemost,* Octobre 2006, vol. 4 (10), 2151-61
- **WERKMEISTER J ; RAMSHAW J.** *Biomed Mater,* Février 2012, vol. 7 (1), 012002
- **NEDDLEMANET WUNSCH.** *J Mol Biol,* 1970, vol. 48, 443